# EUROPEAN PATENT SPECIFICATION

(11) **EP 2 441 840 B1**
(45) Date of publication and mention of the grant of the patent: **06.09.2017**
(21) Application number: 11175326.5
(22) Date of filing: 18.07.2006
(51) Int. Cl.: C12N 15/82, A61K 35/00

(54) **MUCOSAL OR ENTERAL ADMINISTRATION OF BIOLOGICALLY ACTIVE MACROMOLECULES**
MUKOSALE ODER ENTERALE VERABREICHUNG BIOLOGISCH AKTIVER MAKROMOLEKÜLE
ADMINISTRATION MUCOSALE OU ENTERALE DE MACROMOLECULES BIOLOGIQUEMENT ACTIVES

(30) Priority: 18.07.2005 US 699928 P
(43) Date of publication of application: 18.04.2012
(62) Divisional of application: 06766149.6
(73) Proprietor: Protalix Ltd., 20100 Carmiel (IL)
(72) Inventor: SHAALTIEL, Yoseph, 17905 Doar-Na HaMovil (IL); ALMON, Einat, 23840 Timrat (IL)
(74) Representative: Dennemeyer & Associates S.A.

(56) References cited:
- SERENA REGGI ET AL: "Recombinant human acid [beta]-glucosidase stored in tobacco seed is stable, active and taken up by human fibroblasts", PLANT MOLECULAR BIOLOGY, KLUWER ACADEMIC PUBLISHERS, DORDRECHT, NL, vol. 57, no. 1, 1 January 2005 (2005-01-01), pages 101-113, XP019262631, ISSN: 1573-5028, DOI: 10.1007/S11103-004-6832-X
- SIATSKAS C ET AL: "Gene therapy for Fabry disease", JOURNAL OF INHERITED METABOLIC DISEASE, vol. 24, no. Supplement 2, 2001, pages 25-41, XP002670934, & INTERNATIONAL SYMPOSIUM ON LYSOSOMAL STORAGE DISEASES, FABRY DISEASE: NEW INSIGHTS AND FUTURE PERSPE; SEVILLE, SPAIN; APRIL, 2001 ISSN: 0141-8955
- GIDDINGS G ET AL: "Transgenic plants as factories for biopharmaceuticals", NATURE BIOTECHNOLOGY, NATURE PUBLISHING GROUP, NEW YORK, NY, US, vol. 18, no. 11, 1 November 2000 (2000-11-01), pages 1151-1155, XP002988986, ISSN: 1087-0156, DOI: 10.1038/81132
- AZIZ M A ET AL: "Expression of protective antigen in transgenic plants: a step towards edible vaccine against anthrax", BIOCHEMICAL AND BIOPHYSICAL RESEARCH COMMUNICATIONS, ACADEMIC PRESS INC. ORLANDO, FL, US, vol. 299, no. 3, 6 December 2002 (2002-12-06), pages 345-351, XP002314418, ISSN: 0006-291X, DOI: 10.1016/S0006-291X(02)02625-6
- KIM YOUNG-SOOK ET AL: "Expression of the green fluorescent protein (GFP) in tobacco containing low nicotine for the development of edible vaccine", JOURNAL OF PLANT BIOTECHNOLOGY,, vol. 7, no. 2, 1 June 2005 (2005-06-01), pages 97-103, XP002517286,
- HELGA SCHINKEL ET AL: "Production of an active recombinant thrombomodulin derivative in transgenic tobacco plants and suspension cells", TRANSGENIC RESEARCH, KLUWER ACADEMIC PUBLISHERS-PLENUM PUBLISHERS, NE, vol. 14, no. 3, 1 June 2005 (2005-06-01), pages 251-259, XP019269437, ISSN: 1573-9368

### SEQUENCE LISTING

<110> Protalix Ltd. Shaaltiel, Yoseph Almon, Einat
<120> MUCOSAL OR ENTERAL ADMINISTRATION OF BIOLOGICALLY ACTIVE MACROMOLECULES
<130> 31681
<160> 4
<170> PatentIn version 3.3
<210> 1
   <211> 672
   <212> DNA
   <213> Artificial sequence
<220>
   <223> Synthetic codon altered construct coding for the human growth hormone
<400> 1
   atggctactg gatctaggac ttctcttctt cttgctttcg gacttctttg tttgccatgg     60
   cttcaagaag gatctgcttt cccaactatt ccactttcta ggcttttcga taacgctatg    120
   ttgagggctc ataggcttca tcaacttgct ttcgatacct atcaagagtt tgaggaggct    180
   tacattccaa aggagcagaa gtactctttt cttcaaaacc cacaaacctc tctttgtttc    240
   tctgaatcta ttccaactcc atcaaatagg gaggagaccc aacaaaagtc aaaccttgag    300
   cttcttagga tctcacttct tcttattcaa tcttggcttg agccagttca attccttaga    360
   tctgttttcg caaactctct tgtttacgga gcttcagatt caaacgttta cgatcttctt    420
   aaggatcttg aggagggtat tcaaactctt atgggaaggc ttgaagatgg atctccaagg    480
   actggacaaa tcttcaagca gacctactct aagttcgata ccaactctca taacgatgat    540
   gctcttctta agaactacgg acttttgtac tgtttcagga aggatatgga taaggttgag    600
   accttcctta ggatcgttca atgtcgttct gttgaaggat cttgcggatt ctcagagaag    660
   gatgagttgt aa                                                        672
<210> 2
   <211> 223
   <212> PRT
   <213> Artificial sequence
<220>
   <223> Synthetic human growth hormone
<400> 2
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Phe Pro Thr Ile Pro Leu
               20                  25                  30
   Ser Arg Leu Phe Asp Asn Ala Met Leu Arg Ala His Arg Leu His Gln
           35                  40                  45
   Leu Ala Phe Asp Thr Tyr Gln Glu Phe Glu Glu Ala Tyr Ile Pro Lys
       50                  55                  60
   Glu Gln Lys Tyr Ser Phe Leu Gln Asn Pro Gln Thr Ser Leu Cys Phe
   65                  70                  75                  80
   Ser Glu Ser Ile Pro Thr Pro Ser Asn Arg Glu Glu Thr Gln Gln Lys
                   85                  90                  95
   Ser Asn Leu Glu Leu Leu Arg Ile Ser Leu Leu Leu Ile Gln Ser Trp
               100                 105                 110
   Leu Glu Pro Val Gln Phe Leu Arg Ser Val Phe Ala Asn Ser Leu Val
           115                 120                 125
   Tyr Gly Ala Ser Asp Ser Asn Val Tyr Asp Leu Leu Lys Asp Leu Glu
       130                 135                 140
   Glu Gly Ile Gln Thr Leu Met Gly Arg Leu Glu Asp Gly Ser Pro Arg
   145                 150                 155                 160
   Thr Gly Gln Ile Phe Lys Gln Thr Tyr Ser Lys Phe Asp Thr Asn Ser
                   165                 170                 175
   His Asn Asp Asp Ala Leu Leu Lys Asn Tyr Gly Leu Leu Tyr Cys Phe
               180                 185                 190
   Arg Lys Asp Met Asp Lys Val Glu Thr Phe Leu Arg Ile Val Gln Cys
           195                 200                 205
   Arg Ser Val Glu Gly Ser Cys Gly Phe Ser Glu Lys Asp Glu Leu
       210                 215                 220
<210> 3
   <211> 1581
   <212> DNA
   <213> Homo sapiens
<400> 3
   atgaagacta atctttttct ctttctcatc ttttcacttc tcctatcatt atcctcggcc      60
   gaattcgccc gcccctgcat ccctaaaagc ttcggctaca gctcggtggt gtgtgtctgc     120
   aatgccacat actgtgactc ctttgacccc ccgacctttc ctgcccttgg taccttcagc     180
   cgctatgaga gtacacgcag tgggcgacgg atggagctga gtatggggcc catccaggct     240
   aatcacacgg gcacaggcct gctactgacc ctgcagccag aacagaagtt ccagaaagtg     300
   aagggatttg gaggggccat gacagatgct gctgctctca acatccttgc cctgtcaccc     360
   cctgcccaaa atttgctact taaatcgtac ttctctgaag aaggaatcgg atataacatc     420
   atccgggtac ccatggccag ctgtgacttc tccatccgca cctacaccta tgcagacacc     480
   cctgatgatt tccagttgca caacttcagc ctcccagagg aagataccaa gctcaagata     540
   cccctgattc accgagccct gcagttggcc cagcgtcccg tttcactcct tgccagcccc     600
   tggacatcac ccacttggct caagaccaat ggagcggtga atgggaaggg gtcactcaag     660
   ggacagcccg gagacatcta ccaccagacc tgggccagat actttgtgaa gttcctggat     720
   gcctatgctg agcacaagtt acagttctgg gcagtgacag ctgaaaatga gccttctgct     780
   gggctgttga gtggataccc cttccagtgc ctgggcttca cccctgaaca tcagcgagac     840
   ttcattgccc gtgacctagg tcctaccctc gccaacagta ctcaccacaa tgtccgccta     900
   ctcatgctgg atgaccaacg cttgctgctg ccccactggg caaaggtggt actgacagac     960
   ccagaagcag ctaaatatgt tcatggcatt gctgtacatt ggtacctgga ctttctggct    1020
   ccagccaaag ccaccctagg ggagacacac cgcctgttcc ccaacaccat gctctttgcc    1080
   tcagaggcct gtgtgggctc caagttctgg gagcagagtg tgcggctagg ctcctgggat    1140
   cgagggatgc agtacagcca cagcatcatc acgaacctcc tgtaccatgt ggtcggctgg    1200
   accgactgga accttgccct gaaccccgaa ggaggaccca attgggtgcg taactttgtc    1260
   gacagtccca tcattgtaga catcaccaag gacacgtttt acaaacagcc catgttctac    1320
   caccttggcc acttcagcaa gttcattcct gagggctccc agagagtggg gctggttgcc    1380
   agtcagaaga acgacctgga cgcagtggca ctgatgcatc ccgatggctc tgctgttgtg    1440
   gtcgtgctaa accgctcctc taaggatgtg cctcttacca tcaaggatcc tgctgtgggc    1500
   ttcctggaga caatctcacc tggctactcc attcacacct acctgtggca tcgccaagat    1560
   cttttagtcg atactatgta a                                              1581
<210> 4
   <211> 526
   <212> PRT
   <213> Homo sapiens
<400> 4
   Met Lys Thr Asn Leu Phe Leu Phe Leu Ile Phe Ser Leu Leu Leu Ser
   1               5                   10                  15
   Leu Ser Ser Ala Glu Phe Ala Arg Pro Cys Ile Pro Lys Ser Phe Gly
               20                  25                  30
   Tyr Ser Ser Val Val Cys Val Cys Asn Ala Thr Tyr Cys Asp Ser Phe
           35                  40                  45
   Asp Pro Pro Thr Phe Pro Ala Leu Gly Thr Phe Ser Arg Tyr Glu Ser
       50                  55                  60
   Thr Arg Ser Gly Arg Arg Met Glu Leu Ser Met Gly Pro Ile Gln Ala
   65                  70                  75                  80
   Asn His Thr Gly Thr Gly Leu Leu Leu Thr Leu Gln Pro Glu Gln Lys
                   85                  90                  95
   Phe Gln Lys Val Lys Gly Phe Gly Gly Ala Met Thr Asp Ala Ala Ala
               100                 105                 110
   Leu Asn Ile Leu Ala Leu Ser Pro Pro Ala Gln Asn Leu Leu Leu Lys
           115                 120                 125
   Ser Tyr Phe Ser Glu Glu Gly Ile Gly Tyr Asn Ile Ile Arg Val Pro
       130                 135                 140
   Met Ala Ser Cys Asp Phe Ser Ile Arg Thr Tyr Thr Tyr Ala Asp Thr
   145                 150                 155                 160
   Pro Asp Asp Phe Gln Leu His Asn Phe Ser Leu Pro Glu Glu Asp Thr
                   165                 170                 175
   Lys Leu Lys Ile Pro Leu Ile His Arg Ala Leu Gln Leu Ala Gln Arg
           180                 185                 190
   Pro Val Ser Leu Leu Ala Ser Pro Trp Thr Ser Pro Thr Trp Leu Lys
       195                 200                 205
   Thr Asn Gly Ala Val Asn Gly Lys Gly Ser Leu Lys Gly Gln Pro Gly
       210                 215                 220
   Asp Ile Tyr His Gln Thr Trp Ala Arg Tyr Phe Val Lys Phe Leu Asp
   225                 230                 235                 240
   Ala Tyr Ala Glu His Lys Leu Gln Phe Trp Ala Val Thr Ala Glu Asn
                   245                 250                 255
   Glu Pro Ser Ala Gly Leu Leu Ser Gly Tyr Pro Phe Gln Cys Leu Gly
               260                 265                 270
   Phe Thr Pro Glu His Gln Arg Asp Phe Ile Ala Arg Asp Leu Gly Pro
           275                 280                 285
   Thr Leu Ala Asn Ser Thr His His Asn Val Arg Leu Leu Met Leu Asp
       290                 295                 300
   Asp Gln Arg Leu Leu Leu Pro His Trp Ala Lys Val Val Leu Thr Asp
   305                 310                 315                 320
   Pro Glu Ala Ala Lys Tyr Val His Gly Ile Ala Val His Trp Tyr Leu
                   325                 330                 335
   Asp Phe Leu Ala Pro Ala Lys Ala Thr Leu Gly Glu Thr His Arg Leu
               340                 345                 350
   Phe Pro Asn Thr Met Leu Phe Ala Ser Glu Ala Cys Val Gly Ser Lys
           355                 360                 365
   Phe Trp Glu Gln Ser Val Arg Leu Gly Ser Trp Asp Arg Gly Met Gln
       370                 375                 380
   Tyr Ser His Ser Ile Ile Thr Asn Leu Leu Tyr His Val Val Gly Trp
   385                 390                 395                 400
   Thr Asp Trp Asn Leu Ala Leu Asn Pro Glu Gly Gly Pro Asn Trp Val
                   405                 410                 415
   Arg Asn Phe Val Asp Ser Pro Ile Ile Val Asp Ile Thr Lys Asp Thr
               420                 425                 430
   Phe Tyr Lys Gln Pro Met Phe Tyr His Leu Gly His Phe Ser Lys Phe
           435                 440                 445
   Ile Pro Glu Gly Ser Gln Arg Val Gly Leu Val Ala Ser Gln Lys Asn
       450                 455                 460
   Asp Leu Asp Ala Val Ala Leu Met His Pro Asp Gly Ser Ala Val Val
   465                 470                 475                 480
   Val Val Leu Asn Arg Ser Ser Lys Asp Val Pro Leu Thr Ile Lys Asp
                   485                 490                 495
   Pro Ala Val Gly Phe Leu Glu Thr Ile Ser Pro Gly Tyr Ser Ile His
               500                 505                 510
   Thr Tyr Leu Trp His Arg Gln Asp Leu Leu Val Asp Thr Met
           515                 520                 525

## Description

The present invention relates to a unit dosage form for systemic delivery of a biologically active recombinant biomolecule to a subject according to claim 1, and isolated plant cells of the BY2 tobacco cell line for use in the treatment of Fabry disease according to claim 10, as well as isolated plant cells of the BY2 tobacco cell line for use in the treatment of dwarfism.

Innovations in biotechnology have led to a significant increase in the number of protein and peptide therapeutics and other macromolecular drugs. Greater than 84 macromolecules are currently approved for marketing in the United States, and almost 350 more are in clinical development. Further, recent advances in genomic and proteomic technologies are expected to continue to increase the pipeline of macromolecular therapeutic candidates.

Working with macromolecules typically poses a number of challenges, however, that drug developers must overcome in order to successfully develop these compounds into safe and effective therapeutics. For example, proteins and peptides tend to be destroyed by proteolytic enzymes or, in the case of the higher molecular weight proteins, may generate neutralizing antibodies. Moreover, such molecules can exhibit low solubility or poor stability, leading to short shelf lives. As a result, macromolecule therapeutics often quickly lose their effectiveness or require frequent dosing. These factors impact not only cost of therapy, but also patient acceptance and compliance, thus affecting their therapeutic usefulness.

### Oral Administration:

The most common method for protein and peptide-based drug delivery is by invasive methods of drug delivery, such as injections and infusions. Although these are the primary modes for administering macromolecular drugs for systemic diseases, they are also the least desirable for patients and practitioners. The obvious downside of this delivery method is patient acceptance and compliance, limiting most macromolecule development to indications in which the need to use invasive administration routes are not outweighed by associated expenses or inconvenience. As a non-invasive method for systemically delivering drugs, oral administration provides many advantages: ease and convenience of use, access to extensive volume of absorptive surface, high degree of vascularization, relatively lengthy retention time, natural disposal of inactive, non-metabolized ingredients, and more.

Nonetheless, investigations of oral administration of macromolecular pharmaceuticals have not indicated satisfactory levels of efficiency to match the potential of this route. Some of the obstacles are difficulties of ingestion of pills and other solid formulations, lability of biologically active macromolecules in the GI tract, concentration of the biologically active agents at the mucosa, and permeability of GI membranes to biologically active macromolecules.

The oral route of administration of biologically active substances is complicated by both high acidity and enzymatic degradation in the stomach, which can inactivate or destroy biologically active macromolecules before they reach their intended target tissue. Further, effective concentrations of a biologically active macromolecule are difficult to achieve in the large volumes encountered in the GI tract. Thus, to be effective, most drugs must be protected from absorption and/or the environment in the upper GI tract, and then be abruptly released into the intestine or colon. Various strategies are being used in the pharmaceutical industry to overcome the problems associated with oral or enteral administration of therapeutic macromolecules such as proteins. These strategies include covalent linkage with a carrier, coatings and formulations (pH sensitive coatings, polymers and multi-layered coatings, encapsulation, timed release formulations, bioadhesives systems, osmotic controlled delivery systems, etc) designed to slow or prevent release of active ingredients in harsh conditions such as the stomach and upper GI tract. However, preparation of biologically active agents in such formulations requires complex and costly processes. Also employed are mucosal adhesives and penetration enhancers (salicylates, lipid-bile salt-mixed micelles, glycerides, acylcarnitines, etc) for increasing uptake at the mucosa. However, some of these can cause serious local toxicity problems, such as local irritation, abrasion of the epithelial layer and inflammation of tissue. Other strategies to improve oral delivery include mixing the biologically active agent with protease inhibitors, such as aprotinin, soybean trypsin inhibitor, and amastatin; however, enzyme inhibitors are not selective, and also inhibit endogenous macromolecules, causing undesirable side effects. Thus, present methods of oral administration of biologically active molecules cannot ensure efficient delivery of desired biological activity at the target tissue.

### Plant biopharmaceuticals:

Mammalian cells are naturally considered suitable for expression of mammalian genes. However, their use poses many problems: high expense of culturing, and of foremost concern, contamination. Protein xpression obtained with mammalian cells cultures in vitro require very large volumes incurring high costs. The production of recombinant proteins in the milk of transgenic animals (mice, sheep and cows) allows some production costs to be reduced. However, ethical problems and problems of viral and subviral contamination (prions, etc) remain.

Factors in favor of plant systems as sources of animal derived peptides and polypeptides include:, low cost biomass production, low risk of contamination by viruses, pathogens and toxins, the capacity of plant cells to fold and assemble multimeric proteins, in case of oral delivery, low downstream processing requirements as well as reduced ethical problems. Thus, transgenesis of mammalian genes encoding biologically active peptides and/or polypeptides into a plant cell can provide a desirable route for production of new recombinant biologically active molecules in large quantities, at a reduced production cost and without many of the risks of animal cell viral or subviral contamination.

In 1983, several laboratories discovered that it was possible to transfer a heterologous gene into the genome of a plant cell (Bevan et al., 1983; Herrera-Estrella et al., 1983 a and b) and to regenerate transgenic plants from these genetically modified cells. Two transformation approaches are commonly used to produce recombinant pharmaceuticals in plants. In the first, stably transformed transgenic plants are produced using *Agrobacterium-*mediated transformation, particle bombardment, or other standard transformation techniques. *Nicotiana tabacum* is widely used as a model expression system, but other plants have been used, including *Nicotiana bethamiana, Arabidopsis thaliana,* tomato, banana, turnip, black-eyed bean oilseed rape, Ethiopian mustard, potato, rice, wheat, and maize. The second strategy is to infect nontransgenic plants with recombinant viruses that express transgenes during their replication in the host. The two host-virus systems most frequently used are tobacco with tobacco mosaic virus (TMV) or cowpeas with cowpea mosaic virus (CPMV).

Following the initial breakthroughs in plant transgenics, many advances in the production of mammalian recombinant proteins in plant cells and/or transgenic plants have been made. One of the first truly significant results in this field was the production of antibodies in transgenic tobacco plants ("Plantibodies"). A number of diagnostic and therapeutic "plantibodies" are presently becoming available: Tobacco anti-streptococcal secretory IgA (mouse Guy's 13 IgG) against dental caries; diagnostic Alfalfa anti-human IgG - Murine IgG signal peptides - C5-1 (IgG); anti-cancer Wheat and Rice carcinoembryonic antigen - Murine IgG signal peptide; KDEL - ScFvT84.66 (ScFv); anti-cancer Tobacco carcinoembryonic antigen - TMV leader; murine IgG signal peptides; KDEL - T84.66 (IgG) (transiently with Agrobacterium infiltration); Tobacco B-cell lymphoma treatment; idiotype vaccine - Rice a-amylase - 38C13 (scFv), Tobacco anti-colon cancer surface antigen - Murine IgG signal peptide; KDEL - CO17-1A (IgG), and Soybean anti-Herpes simplex virus 2 - Tobacco extensin signal peptide - Anti-HSV-2 (IgG).

In 1990, Sijmons and colleagues expressed the gene of human serum albumin into cells of tobacco and potato. Human serum albumin levels of the order of 0.02 % of the total proteins were obtained in potato leaves, stems and tubers. Other mammalian recombinant proteins that have since been produced in plants include human hemoglobin; antitrypsin, protease; protease inhibitor; collagen and lactoferrin (see US Patent Application Nos: 20030229925 to Legrand et al; 20040072317 to Lenee et al; and 20030096973 to Gruber et al); the hepatitis B surface antigen; interferons; the cholera toxin; human epidermal growth factor (EGF); erythropoietin; h-GM-CSF, and interleukins. In addition, recombinant plant antigens for vaccination and immunization ("Plantigens") are been developed. Several companies already have plant-derived pharmaceuticals available commercially (TrypZean™ and AproliZean™ from Prodigene, Inc.) or in clinical trials [Planet Biotechnology, Inc. (DoxoRx™ and CaroRx™) and Meristem Therapeutics (recombinant human lipase "Meripase®" for CF)].

In most cases, plant-derived biologically active recombinant proteins need to be isolated from the host tissue. Various methods are available for directing the recombinant proteins to specific plant tissues, such as, seeds, leaves, roots, tubers, etc, and organelles such as chloroplasts, in order to achieve high levels of expression, and provide the most convenient plant materials for extraction. Recombinant proteins can also be secreted; however, secreted recombinant proteins (such as secreted "plantibodies") are more prone to degradation than recombinant proteins retained in the plant tissues. It will be appreciated that administration of whole plants or plant cell cultures expressing the recombinant protein has been suggested before (see e.g., recombinant protein expression in Arabidopsis U.S. Pat. Appl. 20030084484). However no experimental data is provided which supports transport of the recombinant protein to the target tissue.

### Plant cell culture:

Plant cell cultures can be used for the production of recombinant peptides and polypeptides. Plant cells can be grown axenically in nutrient medium in bioreactors under controlled conditions, and foreign protein can be harvested from the biomass, or from the culture liquid. Recombinant biologically active macromolecules including antibodies and antibody fragments, enzymes, interleukins, interferons, human hormones, growth factors, blood factors, vaccinesribosome inactivating protein, ricin, and human antitrypsin have been produced in plant cell culture (for a review see Doran, Current Opinion in Biotech 2000; 11; 199-204). Although agricultural systems may provide overall greater yields, in-vitro cell culture offers the advantages of greater ability to manipulate foreign protein levels, shorter overall growth cycle and greater control of the growth environment for regulatory purposes.

Recently, the present inventors have disclosed a unique high yield disposable culture system for plant cells, which has been shown to be effective for the production of biologically active peptides and polypeptides in culture (see PCT IL/2005/000228), demonstrating production of biologically active Human β Interferon, Human Factor X, Human Glucocerebrosidase and Infectious Bursa Disease VPII protein. The recombinant, plant-cell-derived Glucocerebrosidase is currently being evaluated for future use as treatment for Gaucher's Disease.

### Oral administration of plant-derived recombinant biologically active macromolecules:

The use of edible transgenic plants to provide recombinant biologically active agents has been pursued since the early days of agricultural genetic engineering. Transformation of edible leafy crops such as lettuce, cereals such as maize, rice, barley and wheat, legumes such as soybean and pea, and fruits and vegetables such as potato, carrot, tomato and banana has been investigated for efficient delivery of recombinant vaccines. Oral DNA vaccination via edible transgenic plants (corn, potatoes) expressing genes for antigenic proteins has been successfully demonstrated. Mucosal and serum immune responses have been detected against cholera toxin B subunit in mice fed with transgenic potatoes, against LT-B entererotoxin antigen in humans fed with transgenic potato tubers (Mason et al, Vaccine 1998;16:1336-1340), against hepatitis B-surface antigen in humans and mice fed with transgenic lupin and lettuce (Kapusta et al FASEB J, 1999;13:1796-99) and transgenic tomatilla (Gao et al; W Jour Gastroent. 2003;9:996-1002. Multicomponent edible HIV and HBV vaccines have been recently tested in tomato (Shchelkunov, et al, Vestn. Ross.Akad Med Nauk 2004;11:50-55) ; and a multicomponent edible potato vaccine has been recently successfully tested against cholera, rotavirus and ETEC (for a recent review, see Korban et al, J Am Col Nutr 2002; 21:212S-217S). Webster et al (J of Virol,2002;76:7910-12) have reported the successful immunization and boosting of mice against measles with transgenic potato-derived measles virus haemagglutinin protein vaccine. Smart et al (J Immunol 2003; 171: 2116-26) have shown that feeding transgenic lupin expressing sunflower seed antigens can provide mice with protection from experimental asthma.

Various approaches to the preparation and use of transgenic plant material for oral administration have been proposed. Kirk et al (US Patent Application No: 20040175440) disclose the preparation of stable dry homogenates of transgenic plants expressing a heterologous protein, and the oral administration of the homogenates, comprising recombinant fertilization-associated peptides and polypeptides such as zona pellucida glycoprotein, GnRH, LHRH, LH, LDH and anti-sperm antigens, for the induction of effective contraceptive antibodies. Brandle et al (US Patent Application No: 02/137,647 filed May 3, 2002) disclose the production of transgenic plants (in edible and non-food crops) expressing human autoantigens and/or cytokines, as well as methods for the extraction of the biologically active molecules, for the modulation of autoimmune disease, for example Inflammatory Bowel Disease (IBD) and diabetes, by stimulation of oral tolerance to active autoantigens.

However, none of the abovementioned prior art teaches the systemic administration of recombinant peptides and/or polypeptides to the blood circulation and/or to internal organs or organ systems via oral administration of transgenic plant cell culture expressing biologically active recombinant macromolecules.

There is thus a widely recognized need for, and it would be highly advantageous to have, methods and compositions for the enteral (e.g., oral) or mucosal administration of biologically active macromolecules via administration of transgenic plant cells.

SERENA REGGI ET AL, relates to "Recombinant human acid [beta]-glucosidase stored in tobacco seed is stable, active and taken up by human fibroblasts", PLANT MOLECULAR BIOLOGY, KLUWER ACADEMIC PUBLISHERS, DORDRECHT, NL, (20050101), vol. 57, no. 1, pages 101 - 113.

SIATSKAS C ET AL, relates to "Gene therapy for Fabry disease", JOURNAL OF INHERITED METABOLIC DISEASE, & INTERNATIONAL SYMPOSIUM ON LYSOSOMAL STORAGE DISEASES, FABRY DISEASE: NEW INSIGHTS AND FUTURE PERSPE; SEVILLE, SPAIN; APRIL, 2001, (2001), vol. 24, no. Supplement 2.

GIDDINGS G ET AL, relates to "Transgenic plants as factories for biopharmaceuticals", NATURE BIOTECHNOLOGY, NATURE PUBLISHING GROUP, NEW YORK, NY, US, (20001101), vol. 18, no. 11, pages 1151-1155.

AZIZ M A ET AL, relates to "Expression of protective antigen in transgenic plants: a step towards edible vaccine against anthrax", BIOCHEMICAL AND BIOPHYSICAL RESEARCH COMMUNICATIONS, ACADEMIC PRESS INC. ORLANDO, FL, US, (20021206), vol. 299, no. 3, pages 345 - 351.

KIM YOUNG-SOOK ET AL, relates to "Expression of the green fluorescent protein (GFP) in tobacco containing low nicotine for the development of edible vaccine", JOURNAL OF PLANT BIOTECHNOLOGY" (20050601), vol. 7, no. 2, pages 97 -103.

HELGA SCHINKEL ET AL, relates to "Production of an active recombinant thrombomodulin derivative in transgenic tobacco plants and suspension cells", TRANSGENIC RESEARCH, KLUWER ACADEMIC PUBLISHERS-PLENUM PUBLISHERS, NE, (20050601), vol. 14, no. 3, ISSN 1573-9368, pages 251 -259.

### SUMMARY OF THE INVENTION

The present invention relates to a unit dosage form for systemic delivery of a biologically active recombinant biomolecule to a subject, the unit dosage form comprising a therapeutically effective amount of dehydrated isolated plant cells of the BY2 tobacco cell line, said isolated plant cells expressing the recombinant biomolecule.

Preferably, the unit dosage form is formulated for oral administration.

Preferably, the unit dosage form is formulated for mucosal administration.

Preferably, said recombinant biomolecule comprises a recombinant polypeptide.

Preferably, said recombinant polypeptide comprises a lysosomal enzyme.

Preferably, said recombinant polypeptide comprises an alpha galactosidase.

Preferably, said recombinant biomolecule is selected from the group consisting of a chimeric protein, an enzyme, a growth factor, a protease, a lysosomal enzyme, an extra-cellular matrix protein, an infectious viral protein, an antisense oligonucleotide, a dsRNA and a ribozyme.

Preferably, the unit dosage form is selected from the group consisting of a tablet, a pill, a dragee, a capsule, a liquid, a gel, a syrup, a slurry, a suspension.

Preferably, the unit dosage form is a food.

The present invention also relates to isolated plant cells of the BY2 tobacco cell line for use in the treatment of Fabry disease, said isolated plant cells expressing a human alpha galactosidase.

Preferably, isolated plant cells of the BY2 tobacco cell line are for use in the treatment of dwarfism, said isolated plant cells expressing a human growth hormone.

Preferably, the cells are formulated for oral administration.

Preferably, the cells are formulated for mucosal administration.

Preferably, said isolated plant cells comprise dehydrated plant cells.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a bar graph depicting activity of GCD in livers of mice fed with carrot cultures expressing the recombinant protein.
FIG. 2 is a bar graph depicting elevated levels of hGH in hypophysectomized rats administered with hGH expressing BY2 cells as compared to animals administered with naive BY2 cells.
FIGs. 3a-b are bar graphs depicting elevated levels of hGCD in spleen (Figure 3 a) and liver (Figure 3b) of rats orally administered with hGCD expressing plant cells. Peak levels are evident 1 hour following administration.
FIG. 4 is a bar graph depicting elevated levels of FSH in sera of rats orally administered with FSH-expressing plant cells. Peak levels are evident 10 minutes following administration.
FIG. 5 is a photomicrograph showing Western Blot analysis showing GCD levels derived from dry/lyophilized and fresh cell extracts of GCD expressing cells.

### DESCRIPTION OF THE PREFERRED EMBODIMENTS

The present disclosure is of methods and compositions for the mucosal or enteral (e.g., oral) administration of biologically active macromolecules which can be used for diagnostic, cosmetic, prophylactic or therapeutic purposes.

The principles and operation of the present disclosure may be better understood with reference to the drawings and accompanying descriptions.

Before explaining at least one aspect of the disclosure in detail, it is to be understood that the disclosure is not limited in its application to the details set forth in the following description or exemplified by the Examples. The disclosure is capable of other aspects or of being practiced or carried out in various ways. Also, it is to be understood that the phraseology and terminology employed herein is for the purpose of description and should not be regarded as limiting.

Plant-based production of biopharmaceuticals is now in the marketplace and clinical trials for plant-derived therapeutic proteins are underway. However product recovery from plant culture medium or the biomass hampers large-scale commercial use of such recombinant proteins in terms of cost-effectiveness, yield and activity.

Administration of therapeutic proteins such as by following minimal or no further recovery has been previously suggested (see e.g., U.S. Pat. App. 20040175440, 2003013588 and 20030084484). However, to date, no experimental proof has been provided for the actual transport of the recombinant protein to the circulation nor to target organ or tissue (i.e., wherein the target organ is not part of the gastrointestinal tract).

While reducing the present invention to practice, the present inventors uncovered that administration of plant cells expressing the recombinant protein can be used for systemic administration of therapeutic, diagnostic and prophylactic macromolecules (e.g., proteins, oligonucleotide such as antisense and the like).

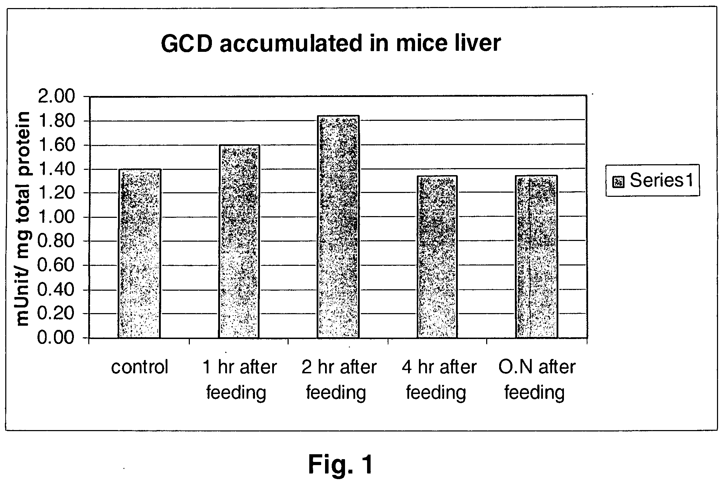

As is illustrated hereinbelow and in the Examples section which follows, the present inventors were able to show that oral administration of carrot cell cultures expressing glucocerebrosidase (GCD) resulted in the accumulation of the active enzyme in livers of mice fed therewith (see Figure 1, Example 1). In addition oral administration of plant cells expressing human growth hormone (hGH), FSH and hGCD to hypophysectomized rats showed successful delivery of the proteins to the circulation and peripheral organs as evidenced by ELISA assay (see Examples 2-4).

Thus, the present disclosure provides for the first time evidence that plant cells can be used as effective carriers for the systemic, mucosal or enteral delivery of biologically active biomolecules such as proteins.

Thus, according to one aspect of the present disclosure there is provided a method of systemically delivering a biologically active, recombinant biomolecule, in a biologically active form, to a subject in need thereof.

The method of this aspect of the present disclosure is effected by expressing the biologically active recombinant biomolecule in plant cells; and enterally (e.g., orally) or mucosally administering to the subject a therapeutically effective amount of the cells expressing the biologically active exogenous recombinant biomolecule, thereby systemically delivering the biologically active recombinant protein, in a biologically active form, to the subject.

As used herein the phrase "systemically delivering" refers to providing organs (internal or external) of the body via the blood circulation.

As used herein the phrase "recombinant biomolecule" refers to a polynucleotide, an oligonucleotide, a polypeptide or a peptide (such as a peptide fragment of a larger polypeptide) which is exogenously expressed (mRNA or protein level) in the plant cells of the present disclosure using recombinant DNA technology.

Examples of proteins which may be recombinantly expressed according to the teachings of the present disclosure include but are not limited to prokaryotic proteins, eukaryotic proteins (e.g., mammalian, plant), chimeric proteins, viral proteins and peptides. Specific examples include, but are not limited to, antibodies (e.g., anti-dental caries), hormones, growth factors, proteases, extra-cellualr matrix proteins (e.g., collagen), enzymes, the infectious bursal disease virus viral protein VPII, Human interferon beta, Human clotting factor, Human factor X, Human lysosomal enzyme, Human glucocerebrosidase, human alpha galactosidase, and Acetyl Choline esterase and high mannose proteins [e.g., Human Cox-2, Human EGF, Human uterine tissue plasminogen activator (tPA), Human DNase I, recombinant gp120, Human tissue plasminogen activator, Human thyroglobulin (hTG), Human CD4 and Human plasminogen)].

It will be appreciated that other biomolecules can be delivered using the teachings of the present disclosure such as oligonucleotides which are involved in gene silencing (e.g., antisense, dsRNA, ribozyme, DNAzyme and the likes).

As used herein the phrase "biological activity" refers to an inherent biological activity (e.g., enzymatic activity, binding activity) of the recombinant protein which is preferably not limited to the elicitation of an immune response for vaccination intentions.

As used herein the phrase "subject in need thereof" refers to a multicellular animal organism (e.g. poultry, e.g., mammal, e.g., human) who may benefit (e.g., clinically aesthetically) from the present disclosure.

As used herein the phrase "plant cells" refers to whole plants, portions thereof (e.g., leaf, root, fruit, seed) or cells isolated therefrom (homogeneous or heterogeneous populations of cells) which express the biologically active recombinant (exogenous) biomolecule.

As used herein the phrase "isolated plant cells" refers to plant cells which are derived from disintegrated plant cell tissue or plant cell cultures.

As used herein the phrase "plant cell culture" refers to any type of native (naturally occurring) plant cells, plant cell lines and genetically modified plant cells, which are not assembled to form a complete plant, such that at least one biological structure of a plant is not present. Optionally, the plant cell culture of this aspect of the present disclosure may comprise a particular type of a plant cell or a plurality of different types of plant cells. It should be noted that optionally plant cultures featuring a particular type of plant cell may be originally derived from a plurality of different types of such plant cells.

Plant cells of the present disclosure comprise an intact cell membrane and/or cell-wall, indicating that no deliberate destruction of these structures is needed prior to administration in order to deliver the bioactive molecule. Thus, preferably at least 30 %, 40 %, 50 %, 60%, 70 %, 80 %, 90 % or 100 % cells administered comprise a substantially intact cell membrane and/or cell-wall.

Plant cells of the present disclosure are derived from a plant (or part thereof), preferably an edible plant, which is amenable to genetic modification so as to express the recombinant protein therein.

Examples of plants which may be used in accordance with this aspect of the present disclosure include, but are not limited to, moss, algae, monocot or dicot, as well as other plants. Examples include, but are not limited to, leafy crops, oil crops, afalfa, tobacco, tomatoes, bananas, carrots, lettuce, maize, cucumber, melon, potatoes grapes and white clover.

The plant cell may optionally be any type of plant cell such as a plant root cell (i.e. a cell derived from, obtained from, or originally based upon, a plant root), more preferably a plant root cell selected from the group consisting of, a celery cell, a ginger cell, a horseradish cell and a carrot cell.

According to presently known preferred configuration of the present disclosure the plant cells are derived from a carrot or from tobacco (see Examples 2-4 of the Examples section below). It will be appreciated that plant cells originating from structures other than roots can be transformed with *Agrobacterium rhizogenes,* inducing hairy root cell development (see, for example, US Patent No. 4,588,693 to Strobel et al), as further described hereibelow. Thus, as described hereinabove, and detailed in the Examples section below, the plant root cell may be an *Agrobacterium rhizogenes* transformed root cell.

Suspension cultures are preferably used in accordance with this aspect of the present disclosure, although callus cultures may also be used.

Expression of the biologically active recombinant protein of this aspect of the present disclosure in cells of the above-described plant cell culture is effected by ligating a nucleic acid sequence expressing same into a nucleic acid construct suitable for plant expression. In addition expression of the biologically active protein of this aspect of the present disclosure in cells of the above-described plant cell culture is effected by ligating a nucleic acid sequence driving the over expression of a plant gene.

Such a nucleic acid construct includes a cis-acting regulatory region such as a promoter sequence for directing transcription of the polynucleotide sequence in the cell in a constitutive or inducible manner. The promoter may be homologous or heterologous to the transformed plant/cell. Or alternatively, such a nucleic acid construct includes an enhancer/promoter element to be inserted into the plant genome in the vicinity to a plant gene (i.e., knock-in).

The promoter may be a plant promoter or a non-plant promoter which is capable of driving high levels of transcription of a linked sequence in the host cell, such as in plant cells and plants. The promoter may be either constitutive or inducible. For example, and not by way of limitation, an inducible promoter can be a promoter that promotes expression or increased expression of the lysosomal enzyme nucleotide sequence after mechanical gene activation (MGA) of the plant, plant tissue or plant cell.

Examples of constitutive plant promoters include, but are not limited to CaMV35S and CaMV19S promoters, FMV34S promoter, sugarcane bacilliform badnavirus promoter, CsVMV promoter, Arabidpsis ACT2/ACT8 actin promoter, Arabidpsis ubiquitin UBQ 1 promoter, barley leaf thionin BTH6 promoter, rice actin promoter, rbcS, the promoter for the chlorophyll a/b binding protein, AdhI, NOS and HMG2, or modifications or derivatives thereof.

An inducible promoter is a promoter induced by a specific stimulus such as stress conditions comprising, for example, light, temperature, chemicals, drought, high salinity, osmotic shock, oxidant conditions or in case of pathogenicity. Usually the promoter is induced before the plant is harvested and as such is referred to as a pre-harvest promoter. Examples of inducible pre-harvest promoters include, but are not limited to, the light-inducible promoter derived from the pea rbcS gene, the promoter from the alfalfa rbcS gene, the promoters DRE, MYC and MYB active in drought; the promoters INT, INPS, prxEa, Ha hsp17.7G4 and RD21 active in high salinity and osmotic stress, and the promoters hsr2O3J and str246C active in pathogenic stress.

The inducible promoter may also be an inducible post-harvest promoter e.g. the inducible MeGA.™ promoter (U.S. Pat. No. 5,689,056). The preferred signal utilized for the rapid induction of the MeGA ™ promoter is a localized wound after the plant has been harvested.

The nucleic acid construct of the present disclosure may also comprise an additional nucleic acid sequence encoding a signal peptide that allows transport of the recombinant protein in-frame fused thereto to a sub-cellular organelle within the plant, as desired. Examples of subcellular organelles of plant cells include, but are not limited to, leucoplasts, chloroplasts, chromoplasts, mitochondria, nuclei, peroxisomes, endoplasmic reticulum and vacuoles.

The expression vectors used for transfecting or transforming the host cells of the disclosure can be additionally modified according to methods known to those skilled in the art to enhance or optimize heterologous gene expression in plants and plant cells. Such modifications include but are not limited to mutating DNA regulatory elements to increase promoter strength or to alter the protein of interest, as well as to optimizing codon usage. Construction of synthetic genes by altering the codon usage is described in for example PCT Patent Application 93/07278.

The nucleic acid construct can be, for example, a plasmid, a bacmid, a phagemid, a cosmid, a phage, a virus or an artificial chromosome. Preferably, the nucleic acid construct of the present disclosure is a plasmid vector, more preferably a binary vector.

The phrase "binary vector" refers to an expression vector which carries a modified T-region from Ti plasmid; enable to be multiplied both in *E. coli* and in *Agrobacterium* cells, and usually comprising reporter gene(s) for plant transformation between the two boarder regions. A binary vector suitable for the present disclosure includes pBI2113, pBI121, pGA482, pGAH, pBIG, pBI101 (Clonetech), pPI (see Example 5 of the Examples section which follows) or modifications thereof.

It will be appreciated that production of active polypeptides in some cases comprises a sequence of events, commencing with expression of the polypeptide which may be followed by post translational modifications, e.g., glycosylation, dimeriztion, methylation and sulfhylation, hydroxylation.

Although plants are capable of glycosylating human proteins at the correct position, the composition of fully processed complex plant glycans differs from mammalian N-linked glycans. Plant glycans, do not have the terminal sialic acid residue or galactose residues common in animal glycans and often contain a xylose or fucose residue with a linkage that is generally not found in mammals (Jenkins et al., 14 Nature Biotech 975-981 (1996); Chrispeels and Faye in transgenic plants pp. 99-114 (Owen, M. and Pen, J. eds. Wiley & Sons, N.Y. 1996; Russell 240 Curr. Top. Microbio. Immunol. (1999).

The nucleic acid construct of the present disclosure can be utilized to stably or transiently transform plant cells. In stable transformation, the nucleic acid molecule of the present disclosure is integrated into the plant genome, and as such it represents a stable and inherited trait. In transient transformation, the nucleic acid molecule is expressed by the cell transformed but not integrated into the genome, and as such represents a transient expression of a specific protein.

There are various methods of introducing foreign genes into both monocotyledonous and dicotyledonous plants (Potrykus, I. (1991). Annu Rev Plant Physiol Plant Mol Biol 42, 205-225; Shimamoto, K. et al. (1989). Fertile transgenic rice plants regenerated from transformed protoplasts. Nature (1989) 338, 274-276).

The principal methods of the stable integration of exogenous DNA into plant genomic DNA include two main approaches:
(i) *Agrobacterium-mediated gene transfer.* See: Klee, H. J. et al. (1987). Annu Rev Plant Physiol 38, 467-486; Klee, H. J. and Rogers, S. G. (1989). Cell Culture and Somatic Cell Genetics of Plants, Vol. 6, Molecular Biology of Plant Nuclear Genes, pp. 2-25, J. Schell and L. K. Vasil, eds., Academic Publishers, San Diego, Cal.; and Gatenby, A. A. (1989). Regulation and Expression of Plant Genes in Microorganisms, pp. 93-112, Plant Biotechnology, S. Kung and C. J. Arntzen, eds., Butterworth Publishers, Boston, Mass. This is especially favored when root cells are used as host cells.
(ii) *Direct DNA uptake.* See, e.g.: Paszkowski, J. et al. (1989). Cell Culture and Somatic Cell Genetics of Plants, Vol. 6, Molecular Biology of Plant Nuclear Genes, pp. 52-68, J. Schell and L. K. Vasil, eds., Academic Publishers, San Diego, Cal.; and Toriyama, K. et al. (1988). Bio/Technol 6, 1072-1074 (methods for direct uptake of DNA into protoplasts). See also: Zhang et al. (1988). Plant Cell Rep 7, 379-384; and Fromm, M. E. et al. (1986). Stable transformation of maize after gene transfer by electroporation. Nature 319, 791-793 (DNA uptake induced by brief electric shock of plant cells). See also: Klein et al. (1988). Bio/Technology 6, 559-563; McCabe, D. E. et al. (1988). Stable transformation of soybean (Glycine max) by particle acceleration. Bio/Technology 6, 923-926; and Sanford, J. C. (1990). Biolistic plant transformation. Physiol Plant 79, 206-209 (DNA injection into plant cells or tissues by particle bombardment). See also: Neuhaus, J. M. et al. (1987). Theor Appl Genet 75, 30-36; and Neuhaus, J. M. and Spangenberg, G. C. (1990). Physiol Plant 79, 213-217 (use of micropipette systems). See U.S. Pat. No. 5,464,765 (glass fibers or silicon carbide whisker transformation of cell cultures, embryos or callus tissue). See also: DeWet, J. M. J. et al. (1985). "Exogenous gene transfer in maize (Zea mays) using DNA-treated pollen," Experimental Manipulation of Ovule Tissue, G. P. Chapman et al., eds., Longman, New York-London, pp. 197-209; and Ohta, Y. (1986). High-Efficiency Genetic Transformation of Maize by a Mixture of Pollen and Exogenous DNA. Proc Natl Acad Sci USA 83, 715-719 (direct incubation of DNA with germinating pollen).

The Agrobacterium-mediated system includes the use of plasmid vectors that contain defined DNA segments which integrate into the plant genomic DNA. Methods of inoculation of the plant tissue vary depending upon the plant species and the Agrobacterium delivery system. A widely used approach is the leaf-disc procedure, which can be performed with any tissue explant that provides a good source for initiation of whole-plant differentiation (Horsch, R. B. et al. (1988). "Leaf disc transformation." Plant Molecular Biology Manual A5, 1-9, Kluwer Academic Publishers, Dordrecht). A supplementary approach employs the Agrobacterium delivery system in combination with vacuum infiltration. The Agrobacterium system is especially useful for in the creation of transgenic dicotyledenous plants.

There are various methods of direct DNA transfer into plant cells. In electroporation, the protoplasts are briefly exposed to a strong electric field, opening up mini-pores to allow DNA to enter. In microinjection, the DNA is mechanically injected directly into the cells using micropipettes. In microparticle bombardment, the DNA is adsorbed on microprojectiles such as magnesium sulfate crystals or tungsten particles, and the microprojectiles are physically accelerated into cells or plant tissues.

Although stable transformation is presently preferred, transient transformation of, for instance, leaf cells, meristematic cells, or the whole plant is also envisaged by the present disclosure. However, in this case measures are taken to exclude viral sequences or selection genes (e.g., antibiotic resistance) for regulatory purposes.

Transient transformation can be effected by any of the direct DNA transfer methods described above or by viral infection using modified plant viruses.

Viruses that have been shown to be useful for the transformation of plant hosts include cauliflower mosaic virus (CaMV), tobacco mosaic virus (TMV), and baculovirus (BV). Transformation of plants using plant viruses is described in, for example: U.S. Pat. No. 4,855,237 (bean golden mosaic virus, BGMV); EPA 67,553 (TMV); Japanese Published Application No. 63-14693 (TMV); EPA 194,809 (BV); EPA 278,667 (BV); and Gluzman, Y. et al. (1988). Communications in Molecular Biology: Viral Vectors, Cold Spring Harbor Laboratory, New York, pp. 172-189. The use of pseudovirus particles in expressing foreign DNA in many hosts, including plants, is described in WO 87/06261.

Construction of plant RNA viruses for the introduction and expression of non-viral exogenous nucleic acid sequences in plants is demonstrated by the above references as well as by: Dawson, W. O. et al. (1989). A tobacco mosaic virus-hybrid expresses and loses an added gene. Virology 172, 285-292; French, R. et al. (1986) Science 231, 1294-1297; and Takamatsu, N. et al. (1990). Production of enkephalin in tobacco protoplasts using tobacco mosaic virus RNA vector. FEBS Lett 269, 73-76.

If the transforming virus is a DNA virus, one skilled in the art may make suitable modifications to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of the DNA will produce the coat protein, which will encapsidate the viral DNA. If the virus is an RNA virus, the virus is generally cloned as a cDNA and inserted into a plasmid. The plasmid is then used to make all of the plant genetic constructs. The RNA virus is then transcribed from the viral sequence of the plasmid, followed by translation of the viral genes to produce the coat proteins which encapsidate the viral RNA.

Construction of plant RNA viruses for the introduction and expression in plants of non-viral exogenous nucleic acid sequences, such as those included in the construct of the present disclosure, is demonstrated in the above references as well as in U.S. Pat. No. 5,316,931.

In one aspect, there is provided for insertion a plant viral nucleic acid, comprising a deletion of the native coat protein coding sequence from the viral nucleic acid, a non-native (foreign) plant viral coat protein coding sequence, and a non-native promoter, preferably the subgenomic promoter of the non-native coat protein coding sequence, and capable of expression in the plant host, packaging of the recombinant plant viral nucleic acid, and ensuring a systemic infection of the host by the recombinant plant viral nucleic acid. Alternatively, the native coat protein coding sequence may be made non-transcribable by insertion of the non-native nucleic acid sequence within it, such that a non-native protein is produced. The recombinant plant viral nucleic acid construct may contain one or more additional non-native subgenomic promoters. Each non-native subgenomic promoter is capable of transcribing or expressing adjacent genes or nucleic acid sequences in the plant host and incapable of recombination with each other and with native subgenomic promoters. In addition, the recombinant plant viral nucleic acid construct may contain one or more *cis*-acting regulatory elements, such as enhancers, which bind a transacting regulator and regulate the transcription of a coding sequence located downstream thereto. Non-native nucleic acid sequences may be inserted adjacent to the native plant viral subgenomic promoter or the native and non-native plant viral subgenomic promoters if more than one nucleic acid sequence is included. The non-native nucleic acid sequences are transcribed or expressed in the host plant under control of the subgenomic promoter(s) to produce the desired products.

In a second aspect, a recombinant plant viral nucleic acid construct is provided as in the first aspectexcept that the native coat protein coding sequence is placed adjacent to one of the non-native coat protein subgenomic promoters instead of adjacent to a non-native coat protein coding sequence.

In a third aspect, a recombinant plant viral nucleic acid construct is provided comprising a native coat protein gene placed adjacent to its subgenomic promoter and one or more non-native subgenomic promoters inserted into the viral nucleic acid construct. The inserted non-native subgenomic promoters are capable of transcribing or expressing adjacent genes in a plant host and are incapable of recombination with each other and with native subgenomic promoters. Non-native nucleic acid sequences may be inserted adjacent to the non-native subgenomic plant viral promoters such that said sequences are transcribed or expressed in the host plant under control of the subgenomic promoters to produce the desired product.

In a fourth aspect, a recombinant plant viral nucleic acid construct is provided as in the third aspectexcept that the native coat protein coding sequence is replaced by a non-native coat protein coding sequence.

Viral vectors are encapsidated by expressed coat proteins encoded by recombinant plant viral nucleic acid constructs as described hereinabove, to produce a recombinant plant virus. The recombinant plant viral nucleic acid construct or recombinant plant virus is used to infect appropriate host plants. The recombinant plant viral nucleic acid construct is capable of replication in a host, systemic spread within the host, and transcription or expression of one or more foreign genes (isolated nucleic acid) in the host to produce the desired protein.

In another aspect, the transformation vehicle comprises viral derived sequences comprising RNA dependent RNA polymerase (RdRp), subgenomic promoter and/or a partial or complete movement protein sequences wherein all the above nucleic acid fragments are cloned into a binary vector. (Gleba etal, Current Opinion in Plant Biology 2004, 7:182-188). In addition to the above, the nucleic acid molecule of the present disclosure can also be introduced into a chloroplast genome thereby enabling chloroplast expression.

A technique for introducing exogenous nucleic acid sequences to the genome of the chloroplasts is known. This technique involves the following procedures. First, plant cells are chemically treated so as to reduce the number of chloroplasts per cell to about one. Then, the exogenous nucleic acid is introduced into the cells preferably via particle bombardment, with the aim of introducing at least one exogenous nucleic acid molecule into the chloroplasts. The exogenous nucleic acid is selected by one ordinarily skilled in the art to be capable of integration into the chloroplast's genome via homologous recombination, which is readily effected by enzymes inherent to the chloroplast. To this end, the exogenous nucleic acid comprises, in addition to a gene of interest, at least one nucleic acid sequence derived from the chloroplast's genome. In addition, the exogenous nucleic acid comprises a selectable marker, which by sequential selection procedures serves to allow an artisan to ascertain that all or substantially all copies of the chloroplast genome following such selection include the exogenous nucleic acid. Further details relating to this technique are found in U.S. Pat. Nos. 4,945,050 and 5,693,507. A polypeptide can thus be produced by the protein expression system of the chloroplast and become integrated into the chloroplast's inner membrane.

Regardless of the method employed, following transformation, plant propagation occurs. In this case micropropagation is effected to include initial tissue culturing; and tissue culture multiplication to obtain enough cells for further use.

Methods of plant cell culturing are well known in the art. Culturing conditions (e.g., culture medium, temperature, gas environment, bioreactor) may be adjusted according to the plant cell used and the expressed protein to achieve optimal expression. Typically, culturing is effected under standard plant cell culture conditions using any conventional plant culture medium. It will be appreciated that plant culture medium includes both aqueous media and dry and concentrated media to which water can be added to produce aqueous media for culturing plant cells (see e.g., U.S. Pat. NOs. 6,020,169 and 6,589,765).

Examples of plant culture media which can be used in accordance with the present disclosure, include, but not limited to, the following well known media: Anderson (Anderson, In Vitro 14:334, 1978; Anderson, Act. Hort., 112:13, 1980), Chee and Pool (Sci. Hort. 32:85, 1987), CLC/Ipomoea (CP) (Chee et al., J. Am. Soc. Hort. Sci. 117:663, 1992), Chu (N.sub.6) (Chu et al., Scientia Sinic. 18:659, 1975; Chu, Proc. Symp. Plant Tiss. Cult., Peking 43, 1978), DCR (Gupta and Durzan, Plant Cell Rep. 4:177, 1985), DKW/Juglans (Driver and Kuniyuki, HortScience 19:507, 1984; McGranahan et al., in: Bonga and Durzan, eds., Cell and Tissue Culture in Forestry, Martinus Nijhoff, Dordrecht, 1987), De Greef and Jacobs (De Greef and Jacobs, Plant Sci. Lett. 17:55, 1979), Eriksson (ER) (Eriksson, Physiol. Plant. 18:976, 1965), Gamborg's B-5 (Gamborg et al., Exp. Cell Res. 50:151, 1968), Gresshoff and Doy (DBM2) (Gresshoff and Doy, Z Pflanzenphysiol. 73:132, 1974), Heller's (Heller, Ann. Sci. Nat. Bot. Biol. Veg. 11th Ser. 14:1, 1953), Hoagland's (Hoagland and Arnon, Circular 347, Calif. Agr. Exp. Stat., Berkeley, 1950), Kao and Michayluk (Kao and Michayluk, Planta 126:105, 1975), Linsmaier and Skoog (Linsmaier and Skoog, Physiol. Plant. 18:100, 1965), Litvay's (LM) (Litvay et al., Plant Cell Rep. 4:325, 1985), McCown's Woody Plant medium (Lloyd and McCown, Proc. Int. Plant Prop. Soc. 30:421, 1981), Murashige and Skoog and various well-known modifications thereof (Murashige and Skoog, Physiol. Plant. 15:473, 1962), Nitsch and Nitsch (Nitsch and Nitsch, Science 163:85, 1969), Quoirin and Lepoivre (Quoirin et al., C. R. Res. Sta. Cult. Fruit Mar., Gembloux 93, 1977), Schenk and Hildebrandt (Schenk and Hildebrandt, Can. J. Bot. 50:199, 1972), White's (White, The Cultivation of Animal and Plant Cells, Ronald Press, NY, 1963), etc. A number of such plant culture media are commercially available from Sigma (St. Louis, Mo.) and other vendors as dry (powdered) media and dry basal salts mixtures, for example.

Preferably culturing is effected using the high yield disposable plant culture device, which has been shown to be effective for the production of biologically active peptides and polypeptides in culture (see PCT IL/2005/000228, which is incorporated herein by reference).

This device, while essentially disposable, is characterized in comprising a reusable harvesting outlet for enabling harvesting of at least a portion of the medium containing cells, thereby enabling the device to be used continuously for one or more subsequent consecutive culturing/harvesting cycles. In an industrial environment, sterility of the harvesting outlet during and after harvesting may be assured to a significantly high degree at relatively low cost, by providing, for example, a sterile hood in which all the necessary connections and disconnections of services to and from the device may be performed. When eventually the device does become contaminated it may then be disposed of with relatively little economic loss. Such devices may be cheaply manufactured, even for production volumes of 50 or 100 liters or more of culture. Further, the ability to perform a number of culturing/harvesting cycles is economically lucrative, lowering even further the effective cost per device.

A battery of such devices can be economically arranged, and the number of devices in the battery may be controlled to closely match production to demand. Thus, the transition from pilot plant bioreactors to large scale production may also be achieved in a relatively simple and economic manner by adding more devices to the battery. Further, the relatively low production volume of each device, coupled with the lack of solid mixers, results in relatively higher yields as compared to typical stainless steel bioreactors.

Thus, culturing of plant cells according to the present disclosure may be effected using a disposable device for axenically culturing and harvesting cells in at least one cycle (as described in length in PCT IL/2005/000228). Such a device comprises a sterilisable disposable container having a top end and a bottom end, which container may be at least partially filled with a suitable sterile biological cell culture medium and/or axenic inoculant and/or sterile air and/or required other sterile additives, the container comprising: (i) a gas outlet for removing excess air and/or waste gases from the container; (ii) an additive inlet for introducing the inoculant and/or the culture medium and/or the additives into the container; and characterized in further comprising (iii) a reusable harvester comprising a flow controller for enabling harvesting of at least a desired portion of the medium containing cells when desired, thereby enabling the device to be used continuously for at least one further consecutive culturing/harvesting cycle, wherein a remainder of the medium containing cells, remaining from a previous harvested cycle, may serve as inoculant for a next culture and harvest cycle, wherein the culture medium and/or the required additives are provided.

Optionally, the disposable container is transparent and/or translucent. Also optionally the device further comprises an air inlet for introducing sterile gas in the form of bubbles into the culture medium through a first inlet opening, wherein the air inlet is connectable to a suitable gas supply. Preferably, the air inlet is for introducing sterile gas more than once during culturing. More preferably, the air inlet is for continuously introducing sterile gas. Optionally, a plurality of different gases are introduced at different times and/or concentrations through the air inlet.

Preferably, the harvester comprising a contamination preventer for substantially preventing introduction of contaminants into the container via the harvester.

Optionally, the container is non-rigid. Preferably, the container is made from a non-rigid plastic material. More preferably, the material is selected from the group comprising polyethylene, polycarbonate, a copolymer of polyethylene and nylon, PVC and EVA.

Optionally, the container is made from a laminate of more than one layer of the materials.

Also optionally, the container is formed by fusion bonding two suitable sheets of the material along predetermined seams.

Preferably, the air inlet comprises an air inlet pipe extending from the inlet opening to a location inside the container at or near the bottom end thereof.

Also preferably, the at least one air inlet comprises a least one air inlet pipe connectable to a suitable air supply and in communication with a plurality of secondary inlet pipes, each the secondary inlet pipe extending to a location inside the container, via a suitable inlet opening therein, for introducing sterile air in the form of bubbles into the culture medium. More preferably, the device comprises a substantially box-like geometrical configuration, having an overall length, height and width. Most preferably, the height-to-length ratio is between about 1 and about 3, and preferably about 1.85. Optionally, the height to width ratio is between about 5 and about 30, and preferably about 13.

Preferably, the device comprises a support aperture substantially spanning the depth of the device, the aperture adapted to enable the device to be supported on a suitable pole support.

Optionally, the device further comprises a support structure for supporting the device. Preferably, the support structure comprises a pair of opposed frames, each of the frames comprising upper and lower support members spaced by a plurality of substantially parallel vertical support members suitably joined to the upper and lower support members. More preferably, the plurality of vertical support members consists of at least one the vertical support member at each longitudinal extremity of the upper and lower support members.

Also more preferably, the frames are spaced from each other by a plurality of spacing bars releasably or integrally joined to the frames.

Also more preferably, the spacing bars are strategically located such that the device may be inserted and removed relatively easily from the support structure.

Optionally, the lower support member of each the frame comprises at least one lower support adapted for receiving and supporting a corresponding portion of the bottom end of the device.

Preferably, each the lower support is in the form of suitably shaped tab projecting from each of the lower support members in the direction of the opposed frame.

Optionally, the frames each comprise at least one interpartitioner projecting from each frame in the direction of the opposed frame, for to pushing against the sidewall of the device at a predetermined position, such that opposed pairs of the interpartitioner effectively reduce the width of the device at the predetermined position.

Preferably, the interpartitioner comprises suitable substantially vertical members spaced from the upper and lower support members in a direction towards the opposed frame with suitable upper and lower struts.

Optionally, the support structure may comprise a plurality of castors for transporting the devices.

Optionally, at least some of the air bubbles comprise a mean diameter of between about 1 mm and about 10 mm.

Also optionally, at least some of the air bubbles comprise a mean diameter of about 4 mm.

Optionally, the container comprises a suitable filter mounted on the gas outlet for substantially preventing introduction of contaminants into the container via the gas outlet.

Preferably, the container further comprises a suitable filter mounted on the additive inlet for substantially preventing introduction of contaminants into the container via the additive inlet.

Also preferably, there is a contamination preventer which comprises a U-shaped fluid trap, wherein one arm thereof is aseptically mounted to an external outlet of the harvester by suitable aseptic connector.

Preferably, the harvester is located at the bottom of the bottom end of the container.

Also preferably, the harvester is located near the bottom of the bottom end of the container, such that at the end of each harvesting cycle the remainder of the medium containing cells automatically remains at the bottom end of the container up to a level below the level of the harvester.

Optionally and preferably, the remainder of the medium containing cells is determined at least partially according to a distance d2 from the bottom of the container to the harvester.

Preferably, the remainder of the medium containing cells comprises from about 0.5% to about 45% of the original volume of the culture medium and the inoculant. More preferably, the remainder of the medium containing cells comprises from about 10% to about 20% of the original volume of the culture medium and the inoculant. More preferably, the remainder of the medium containing cells comprises from about 0.5%- 2% original volume of the culture medium and the inoculant.

Optionally, the bottom end is substantially convex.

Also optionally, the bottom end is substantially frusta-conical.

Preferably, the container comprises an internal fillable volume of between about 5 liters and about 200 liters, preferably between about 50 liters and 150 liters, and preferably about 100 liters.

Optionally, the device further comprises suitable attacher for attaching the device to a suitable support structure. Preferably, the attacher comprises a loop of suitable material preferably integrally attached to the top end of the container.

Once plant cells expressing the above-described recombinant protein are obtained, they are administered to the subject.

Cells of the present disclosure can be administered to the subject per se (e.g., suspension or de-hydrated) or in a pharmaceutical composition where they are mixed with suitable carriers or excipients.

As used herein, a "pharmaceutical composition" refers to a preparation of one or more of the active ingredients described herein with other chemical components such as physiologically suitable carriers and excipients. The purpose of a pharmaceutical composition is to facilitate administration of a compound to an organism.

As used herein, the term "active ingredient" refers to the recombinant protein expressing cells accountable for the intended biological effect. As demonstrated in Example 5 of the Examples section which follows, cells of the present disclosure may be dehydrated (e.g., comprise less than 10 % water) as they still maintain biological activity upon dehydration. Preferably the recombinant biomolecule is not zona pellucida glycoprotein, GnRH, LHRH, LH and LDH in dehydrated cell preparations.

Hereinafter, the phrases "physiologically acceptable carrier" and "pharmaceutically acceptable carrier," which may be used interchangeably, refer to a carrier or a diluent that does not cause significant irritation to an organism and does not abrogate the biological activity and properties of the administered compound. An adjuvant is included under these phrases. Preferably the carrier used is a non-immunogenic carrier and further preferably does not stimulate the gut associated lymphatic tissue.

Herein, the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils, and polyethylene glycols.

Techniques for formulation and administration of drugs may be found in the latest edition of "Remington's Pharmaceutical Sciences," Mack Publishing Co., Easton, PA, which is herein fully incorporated by reference.

Suitable routes of administration may, for example, include mucosal and enteral.

As used herein the phrase "enteral administration" refers to administration through any part of the gastro-intestinal tract, such as rectal administration, colonic administration, intestinal administration (proximal or distal) and gastric administration. Preferably, enteral administration refers to oral administration.

Pharmaceutical compositions for use in accordance with the present disclosure thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries as desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, and sodium carbomethylcellulose; and/or physiologically acceptable polymers such as polyvinylpyrrolidone (PVP). If desired, disintegrating agents, such as cross-linked polyvinyl pyrrolidone, agar, or alginic acid or a salt thereof, such as sodium alginate, may be added.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions that can be used orally include push-fit capsules made of gelatin, as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate, and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

Examples of mucosal delivery include but are not limited to mouth delivery, pharynx delivery, esophagus delivery, rectal delivery and vaginal delivery.

Preferably, the site of mucosal delivery is via the mouth. Mucosal delivery via the mouth may be affected by sublingual delivery, which is systemic delivery of active agents through the mucosal membranes lining the floor of the mouth or buccal delivery, which is agent administration through the mucosal membranes lining the cheeks (buccal mucosa). Formulations which are particularly useful for mouth mucosal delivery include, but are not limited to mouthwashes, strips, foams, chewing gums, oral sprays, lozenges, foods, toothpaste and capsules. A particularly preferred formulation is a chewing gum.

The formulations, e.g. chewing gums can be low or high moisture, sugar or sugarless, wax containing or wax free, low calorie (via high base or low calorie bulking agents), and/or may contain dental agents. It will be appreciated that in this case, due to the mechanical disruption of the membrane and/or wall of the cells administered, the active ingredient of the present disclosure can be released to the mouth and act locally (such as for treating or diagnosing dental caries).

The active agents (i.e., plant cells) of the present disclosure may also be encapsulated or entrapped to give a delayed release from the mucosal formulations. Any standard technique which gives partial or full encapsulation of the active agents can be used. These techniques include, but are not limited to, spray drying, spray chilling, fluid-bed coating and coacervation. These encapsulation techniques may be used individually in a single step process or in any combination in a multiple step process.

Other methods of providing delayed release formulations include, but are not limited to agglomeration to give partial encapsulation, fixation or absorption which also gives partial encapsulation, and entrapment into an extruded compound.

The amount of coating or encapsulating material on the active agent also may control the length of time for its release from chewing gum.

Generally, the higher the level of coating and the lower the amount of active agent, the slower the release. Methods and materials for formulating delayed release formulations are known in the art. Example, PCT Pat. App. publication No. WO 00/35298 teaches methods and materials for formulating delayed release formulations in chewing gums.

The active agents of the present disclosure (i.e. active agents derived from a particular fruit cell culture) may be formulated in different ways and administered via the same vehicle. For example, the active agents could be encapsulated for fast release, moderate release, and slow release in the same vehicle. Furthermore the active agents of the present disclosure may be added to a gum coating for fast release and also added to the gum center with or without encapsulation for slow release.

Faster absorption may be affected by increasing flavor levels as well as the addition of other flavor components, such as menthol and menthol derivatives, limonene, carvone, isomenthol, eucalyptol, menthone, pynene, camphor and camphor derivatives, as well as monoterpene natural products, monoterpene derivatives, and sesquaterpenes, including caryophyllene and copaene.

The formulations may include other agents which enhance the penetration of the active agents through the mucous and into the blood. Examples of such agents include, but are not limited to 23-lauryl ether, Aprotinin, Azone, Benzalkonium chloride, Cetylpyridinium chloride, Cetyltrimethylammonium bromide, Cyclodextrin, Dextran sulfate, Lauric acid, Lauric acid/Propylene glycol, Lysophosphatidylcholine, Menthol, Methoxysalicylate, Methyloleate, Oleic acid, Phosphatidylcholine, Polyoxyethylene, Polysorbate 80, Sodium EDTA, Sodium glycocholate, Sodium glycodeoxycholate, Sodium lauryl sulfate, Sodium salicylate, Sodium taurocholate, Sodium taurodeoxycholate, Sulfoxides and various alkyl glycosides.

Other modifications may also affect the release rate of the active agents into the mucosa. Texture modifiers to soften base may give faster release where hard bases may give slower release. Addition of alkaline materials such as sodium bicarbonate or sodium hydroxide may make the saliva slightly alkaline, which may increase buccal/lingual absorption of the medicament into the bloodstream.

Release of the active agents of the present disclosure may also be affected by the shape and size of the formulation. For example, flat stick pieces of gum with large surface area may release actives faster into saliva from gum when chewed, whereas round or cube pieces may release medicaments and actives more slowly.

Tableting of chewing gum is disclosed in U.K. Patent Publication No. 1,489,832; U.S. Patent No. 4,753,805; EP Patent Publication No. 0 221 850; and Italy Patent Publication No. 1,273,487. These patents disclose active agents added to chewing gum which is then tableted.

Coloring agents may also be added to the formulations. Coloring agents contemplated by the present disclosure include food quality dyes. Film formers preferably added to the syrup include methyl cellulose, gelatins, hydroxypropyl cellulose, ethyl cellulose, hydroxyethyl cellulose, carboxymethyl cellulose and the like and combinations thereof. According to a preferred aspect, fruit cell cultures of the present disclosure are provided in a non-coloring concentration.

It should be noted that plant cells expressing the recombinant biomolecule of the present disclosure may be formulated in a unit dosage form (such as an oral unit dosage form).

Determination of a therapeutically effective amount is well within the capability of those skilled in the art, especially in light of the detailed disclosure provided herein.

For any preparation used in the methods of the disclosure, the dosage or the therapeutically effective amount can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration, and dosage can be chosen by the individual physician in view of the patient's condition. (See, e.g., Fingl, E. et al. (1975), "The Pharmacological Basis of Therapeutics," Ch. 1, p.1.)

Dosage amount and administration intervals may be adjusted individually to provide sufficient plasma or brain levels of the active ingredient to induce or suppress the biological effect (i.e., minimally effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks, or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present disclosure may, if desired, be presented in a pack or dispenser device, such as an FDA-approved kit, which may contain one or more unit dosage forms containing the active ingredient. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation of the disclosure formulated in a pharmaceutically acceptable carrier may also be prepared, placed in an appropriate container, and labeled for treatment of an indicated condition, as further detailed above.

Recombinant proteins administered as described can find numerous uses in therapy, diagnostics and cosmetics.

Thus, for example, the above described teachings can be used to treat any disease (i.e., chronic or acute) or condition in which administration of the biomolecule of the present disclosure may be therapeutically beneficial. For example, the present inventors have shown accumulation of GCD in livers of mice fed with the enzyme suggesting its use in the treatment of Gaucher's disease.

Examples of other diseases which may be treated using the teachings of the present disclosure include, but are not limited to:
***Inflammatory diseases** -* Include, but are not limited to, chronic inflammatory diseases and acute inflammatory diseases.
***Inflammatory diseases associated with hypersensitivity***

Examples of hypersensitivity include, but are not limited to, Type I hypersensitivity, Type II hypersensitivity, Type III hypersensitivity, Type IV hypersensitivity, immediate hypersensitivity, antibody mediated hypersensitivity, immune complex mediated hypersensitivity, T lymphocyte mediated hypersensitivity and DTH.

Type I or immediate hypersensitivity, such as asthma.

Type II hypersensitivity include, but are not limited to, rheumatoid diseases, rheumatoid autoimmune diseases, rheumatoid arthritis (Krenn V. et al., Histol Histopathol 2000 Jul;15 (3):791), spondylitis, ankylosing spondylitis (Jan Voswinkel et al., Arthritis Res 2001; 3 (3): 189), systemic diseases, systemic autoimmune diseases, systemic lupus erythematosus (Erikson J. et al., Immunol Res 1998; 17 (1-2):49), sclerosis, systemic sclerosis (Renaudineau Y. et al., Clin Diagn Lab Immunol. 1999 Mar;6 (2):156); Chan OT. et al., Immunol Rev 1999 Jun;169:107), glandular diseases, glandular autoimmune diseases, pancreatic autoimmune diseases, diabetes, Type I diabetes (Zimmet P. Diabetes Res Clin Pract 1996 Oct;34 Suppl:S125), thyroid diseases, autoimmune thyroid diseases, Graves' disease (Orgiazzi J. Endocrinol Metab Clin North Am 2000 Jun;29 (2):339), thyroiditis, spontaneous autoimmune thyroiditis (Braley-Mullen H. and Yu S, J Immunol 2000 Dec 15;165 (12):7262), Hashimoto's thyroiditis (Toyoda N. et al., Nippon Rinsho 1999 Aug;57 (8):1810), myxedema, idiopathic myxedema (Mitsuma T. Nippon Rinsho. 1999 Aug;57 (8):1759); autoimmune reproductive diseases, ovarian diseases, ovarian autoimmunity (Garza KM. et al., J Reprod Immunol 1998 Feb;37 (2):87), autoimmune anti-sperm infertility (Diekman AB. et al., Am J Reprod Immunol. 2000 Mar;43 (3):134), repeated fetal loss (Tincani A. et al., Lupus 1998;7 Suppl 2:S107-9), neurodegenerative diseases, neurological diseases, neurological autoimmune diseases, multiple sclerosis (Cross AH. et al., J Neuroimmunol 2001 Jan 1;112 (1-2): 1), Alzheimer's disease (Oron L. et al., J Neural Transm Suppl. 1997;49:77), myasthenia gravis (Infante AJ. And Kraig E, Int Rev Immunol 1999; 18 (1-2):83), motor neuropathies (Kornberg AJ. J Clin Neurosci. 2000 May;7 (3):191), Guillain-Barre syndrome, neuropathies and autoimmune neuropathies (Kusunoki S. Am J Med Sci. 2000 Apr;319 (4):234), myasthenic diseases, Lambert-Eaton myasthenic syndrome (Takamori M. Am J Med Sci. 2000 Apr;319 (4):204), paraneoplastic neurological diseases, cerebellar atrophy, paraneoplastic cerebellar atrophy, non-paraneoplastic stiff man syndrome, cerebellar atrophies, progressive cerebellar atrophies, encephalitis, Rasmussen's encephalitis, amyotrophic lateral sclerosis, Sydeham chorea, Gilles de la Tourette syndrome, polyendocrinopathies, autoimmune polyendocrinopathies (Antoine JC. and Honnorat J. Rev Neurol (Paris) 2000 Jan;156 (1):23); neuropathies, dysimmune neuropathies (Nobile-Orazio E. et al., Electroencephalogr Clin Neurophysiol Suppl 1999;50:419); neuromyotonia, acquired neuromyotonia, arthrogryposis multiplex congenita (Vincent A. et al., Ann N Y Acad Sci. 1998 May 13;841:482), cardiovascular diseases, cardiovascular autoimmune diseases, atherosclerosis (Matsuura E. et al., Lupus. 1998;7 Suppl 2:S135), myocardial infarction (Vaarala O. Lupus. 1998;7 Suppl 2:S132), thrombosis (Tincani A. et al., Lupus 1998;7 Suppl 2:S107-9), granulomatosis, Wegener's granulomatosis, arteritis, Takayasu's arteritis and Kawasaki syndrome (Praprotnik S. et al., Wien Klin Wochenschr 2000 Aug 25;112 (15-16):660); anti-factor VIII autoimmune disease (Lacroix-Desmazes S. et al., Semin Thromb Hemost.2000;26 (2):157); vasculitises, necrotizing small vessel vasculitises, microscopic polyangiitis, Churg and Strauss syndrome, glomerulonephritis, pauci-immune focal necrotizing glomerulonephritis, crescentic glomerulonephritis (Noel LH. Ann Med Interne (Paris). 2000 May; 151 (3):178); antiphospholipid syndrome (Flamholz R. et al., J Clin Apheresis 1999;14 (4):171); heart failure, agonist-like beta-adrenoceptor antibodies in heart failure (Wallukat G. et al., Am J Cardiol. 1999 Jun 17;83 (12A):75H), thrombocytopenic purpura (Moccia F. Ann Ital Med Int. 1999 Apr-Jun;14 (2):114); hemolytic anemia, autoimmune hemolytic anemia (Efremov DG. et al., Leuk Lymphoma 1998 Jan;28 (3-4):285), gastrointestinal diseases, autoimmune diseases of the gastrointestinal tract, intestinal diseases, chronic inflammatory intestinal disease (Garcia Herola A. et al., Gastroenterol Hepatol. 2000 Jan;23 (1):16), celiac disease (Landau YE. and Shoenfeld Y. Harefuah 2000 Jan 16;138 (2):122), autoimmune diseases of the musculature, myositis, autoimmune myositis, Sjogren's syndrome (Feist E. et al., Int Arch Allergy Immunol 2000 Sep;123 (1):92); smooth muscle autoimmune disease (Zauli D. et al., Biomed Pharmacother 1999 Jun;53 (5-6):234), hepatic diseases, hepatic autoimmune diseases, autoimmune hepatitis (Manns MP. J Hepatol 2000 Aug;33 (2):326) and primary biliary cirrhosis (Strassburg CP. et al., Eur J Gastroenterol Hepatol. 1999 Jun;11 (6):595).

Type IV or T cell mediated hypersensitivity, include, but are not limited to, rheumatoid diseases, rheumatoid arthritis (Tisch R, McDevitt HO. Proc Natl Acad Sci U S A 1994 Jan 18;91 (2):437), systemic diseases, systemic autoimmune diseases, systemic lupus erythematosus (Datta SK., Lupus 1998;7 (9):591), glandular diseases, glandular autoimmune diseases, pancreatic diseases, pancreatic autoimmune diseases, Type 1 diabetes (Castano L. and Eisenbarth GS. Ann. Rev. Immunol. 8:647); thyroid diseases, autoimmune thyroid diseases, Graves' disease (Sakata S. et al., Mol Cell Endocrinol 1993 Mar;92 (1):77); ovarian diseases (Garza KM. et al., J Reprod Immunol 1998 Feb;37 (2):87), prostatitis, autoimmune prostatitis (Alexander RB. et al., Urology 1997 Dec;50 (6):893), polyglandular syndrome, autoimmune polyglandular syndrome, Type I autoimmune polyglandular syndrome (Hara T. et al., Blood. 1991 Mar 1;77 (5):1127), neurological diseases, autoimmune neurological diseases, multiple sclerosis, neuritis, optic neuritis (Soderstrom M. et al., J Neurol Neurosurg Psychiatry 1994 May;57 (5):544), myasthenia gravis (Oshima M. et al., Eur J Immunol 1990 Dec;20 (12):2563), stiff-man syndrome (Hiemstra HS. et al., Proc Natl Acad Sci U S A 2001 Mar 27;98 (7):3988), cardiovascular diseases, cardiac autoimmunity in Chagas' disease (Cunha-Neto E. et al., J Clin Invest 1996 Oct 15;98 (8):1709), autoimmune thrombocytopenic purpura (Semple JW. et al., Blood 1996 May 15;87 (10):4245), anti-helper T lymphocyte autoimmunity (Caporossi AP. et al., Viral Immunol 1998;11 (1):9), hemolytic anemia (Sallah S. et al., Ann Hematol 1997 Mar;74 (3):139), hepatic diseases, hepatic autoimmune diseases, hepatitis, chronic active hepatitis (Franco A. et al., Clin Immunol Immunopathol 1990 Mar;54 (3):382), biliary cirrhosis, primary biliary cirrhosis (Jones DE. Clin Sci (Colch) 1996 Nov;91 (5):551), nephric diseases, nephric autoimmune diseases, nephritis, interstitial nephritis (Kelly CJ. J Am Soc Nephrol 1990 Aug;1 (2):140), connective tissue diseases, ear diseases, autoimmune connective tissue diseases, autoimmune ear disease (Yoo TJ. et al., Cell Immunol 1994 Aug;157 (1):249), disease of the inner ear (Gloddek B. et al., Ann N Y Acad Sci 1997 Dec 29;830:266), skin diseases, cutaneous diseases, dermal diseases, bullous skin diseases, pemphigus vulgaris, bullous pemphigoid and pemphigus foliaceus.

Examples of delayed type hypersensitivity include, but are not limited to, contact dermatitis and drug eruption.

Examples of types of T lymphocyte mediating hypersensitivity include, but are not limited to, helper T lymphocytes and cytotoxic T lymphocytes.

Examples of helper T lymphocyte-mediated hypersensitivity include, but are not limited to, Tₕ1 lymphocyte mediated hypersensitivity and Tₕ2 lymphocyte mediated hypersensitivity.

### Autoimmune diseases

Include, but are not limited to, cardiovascular diseases, rheumatoid diseases, glandular diseases, gastrointestinal diseases, cutaneous diseases, hepatic diseases, neurological diseases, muscular diseases, nephric diseases, diseases related to reproduction, connective tissue diseases and systemic diseases.

Examples of autoimmune cardiovascular diseases include, but are not limited to atherosclerosis (Matsuura E. et al., Lupus. 1998;7 Suppl 2:S135), myocardial infarction (Vaarala O. Lupus. 1998;7 Suppl 2:S132), thrombosis (Tincani A. et al., Lupus 1998;7 Suppl 2:S107-9), Wegener's granulomatosis, Takayasu's arteritis, Kawasaki syndrome (Praprotnik S. et al., Wien Klin Wochenschr 2000 Aug 25;112 (15-16):660), anti-factor VIII autoimmune disease (Lacroix-Desmazes S. et al., Semin Thromb Hemost.2000;26 (2):157), necrotizing small vessel vasculitis, microscopic polyangiitis, Churg and Strauss syndrome, pauci-immune focal necrotizing and crescentic glomerulonephritis (Noel LH. Ann Med Interne (Paris). 2000 May;151 (3):178), antiphospholipid syndrome (Flamholz R. et al., J Clin Apheresis 1999;14 (4):171), antibody-induced heart failure (Wallukat G. et al., Am J Cardiol. 1999 Jun 17;83 (12A):75H), thrombocytopenic purpura (Moccia F. Ann Ital Med Int. 1999 Apr-Jun;14 (2):114; Semple JW. et al., Blood 1996 May 15;87 (10):4245), autoimmune hemolytic anemia (Efremov DG. et al., Leuk Lymphoma 1998 Jan;28 (3-4):285; Sallah S. et al., Ann Hematol 1997 Mar;74 (3):139), cardiac autoimmunity in Chagas' disease (Cunha-Neto E. et al., J Clin Invest 1996 Oct 15;98 (8):1709) and anti-helper T lymphocyte autoimmunity (Caporossi AP. et al., Viral Immunol 1998;11 (1):9).

Examples of autoimmune rheumatoid diseases include, but are not limited to rheumatoid arthritis (Krenn V. et al., Histol Histopathol 2000 Jul;15 (3):791; Tisch R, McDevitt HO. Proc Natl Acad Sci units S A 1994 Jan 18;91 (2):437) and ankylosing spondylitis (Jan Voswinkel et al., Arthritis Res 2001; 3 (3): 189).

Examples of autoimmune glandular diseases include, but are not limited to, pancreatic disease, Type I diabetes, thyroid disease, Graves' disease, thyroiditis, spontaneous autoimmune thyroiditis, Hashimoto's thyroiditis, idiopathic myxedema, ovarian autoimmunity, autoimmune anti-sperm infertility, autoimmune prostatitis and Type I autoimmune polyglandular syndrome. Diseases include, but are not limited to autoimmune diseases of the pancreas, Type 1 diabetes (Castano L. and Eisenbarth GS. Ann. Rev. Immunol. 8:647; Zimmet P. Diabetes Res Clin Pract 1996 Oct;34 Suppl:S125), autoimmune thyroid diseases, Graves' disease (Orgiazzi J. Endocrinol Metab Clin North Am 2000 Jun;29 (2):339; Sakata S. et al., Mol Cell Endocrinol 1993 Mar;92 (1):77), spontaneous autoimmune thyroiditis (Braley-Mullen H. and Yu S, J Immunol 2000 Dec 15;165 (12):7262), Hashimoto's thyroiditis (Toyoda N. et al., Nippon Rinsho 1999 Aug;57 (8):1810), idiopathic myxedema (Mitsuma T. Nippon Rinsho. 1999 Aug;57 (8):1759), ovarian autoimmunity (Garza KM. et al., J Reprod Immunol 1998 Feb;37 (2):87), autoimmune anti-sperm infertility (Diekman AB. et al., Am J Reprod Immunol. 2000 Mar;43 (3):134), autoimmune prostatitis (Alexander RB. et al., Urology 1997 Dec;50 (6):893) and Type I autoimmune polyglandular syndrome (Hara T. et al., Blood. 1991 Mar 1;77 (5):1127).

Examples of autoimmune gastrointestinal diseases include, but are not limited to, chronic inflammatory intestinal diseases (Garcia Herola A. et al., Gastroenterol Hepatol. 2000 Jan; 23 (1):16), celiac disease (Landau YE. and Shoenfeld Y. Harefuah 2000 Jan 16; 138 (2):122), colitis, ileitis and Crohn's disease.

Examples of autoimmune cutaneous diseases include, but are not limited to, autoimmune bullous skin diseases, such as, but are not limited to, pemphigus vulgaris, bullous pemphigoid and pemphigus foliaceus.

Examples of autoimmune hepatic diseases include, but are not limited to, hepatitis, autoimmune chronic active hepatitis (Franco A. et al., Clin Immunol Immunopathol 1990 Mar; 54 (3):382), primary biliary cirrhosis (Jones DE. Clin Sci (Colch) 1996 Nov; 91 (5):551; Strassburg CP. et al., Eur J Gastroenterol Hepatol. 1999 Jun; 11 (6):595) and autoimmune hepatitis (Manns MP. J Hepatol 2000 Aug; 33 (2):326).

Examples of autoimmune neurological diseases include, but are not limited to, multiple sclerosis (Cross AH. et al., J Neuroimmunol 2001 Jan 1;112 (1-2):1), Alzheimer's disease (Oron L. et al., J Neural Transm Suppl. 1997;49:77), myasthenia gravis (Infante AJ. And Kraig E, Int Rev Immunol 1999; 18 (1-2):83; Oshima M. et al, Eur J Immunol 1990 Dec; 20 (12):2563), neuropathies, motor neuropathies (Kornberg AJ. J Clin Neurosci. 2000 May;7 (3):191); Guillain-Barre syndrome and autoimmune neuropathies (Kusunoki S. Am J Med Sci. 2000 Apr;319 (4):234), myasthenia, Lambert-Eaton myasthenic syndrome (Takamori M. Am J Med Sci. 2000 Apr;319 (4):204); paraneoplastic neurological diseases, cerebellar atrophy, paraneoplastic cerebellar atrophy and stiff-man syndrome (Hiemstra HS. et al., Proc Natl Acad Sci units S A 2001 Mar 27;98 (7):3988); non-paraneoplastic stiff man syndrome, progressive cerebellar atrophies, encephalitis, Rasmussen's encephalitis, amyotrophic lateral sclerosis, Sydeham chorea, Gilles de la Tourette syndrome and autoimmune polyendocrinopathies (Antoine JC. and Honnorat J. Rev Neurol (Paris) 2000 Jan;156 (1):23); dysimmune neuropathies (Nobile-Orazio E. et al., Electroencephalogr Clin Neurophysiol Suppl 1999;50:419); acquired neuromyotonia, arthrogryposis multiplex congenita (Vincent A. et al., Ann N Y Acad Sci. 1998 May 13;841:482), neuritis, optic neuritis (Soderstrom M. et al., J Neurol Neurosurg Psychiatry 1994 May;57 (5):544) and neurodegenerative diseases.

Examples of autoimmune muscular diseases include, but are not limited to, myositis, autoimmune myositis and primary Sjogren's syndrome (Feist E. et al., Int Arch Allergy Immunol 2000 Sep; 123 (1):92) and smooth muscle autoimmune disease (Zauli D. et al., Biomed Pharmacother 1999 Jun;53 (5-6):234).

Examples of autoimmune nephric diseases include, but are not limited to, nephritis and autoimmune interstitial nephritis (Kelly CJ. J Am Soc Nephrol 1990 Aug; 1 (2):140).

Examples of autoimmune diseases related to reproduction include, but are not limited to, repeated fetal loss (Tincani A. et al., Lupus 1998; 7 Suppl 2:S107-9).

Examples of autoimmune connective tissue diseases include, but are not limited to, ear diseases, autoimmune ear diseases (Yoo TJ. et al., Cell Immunol 1994 Aug; 157 (1):249) and autoimmune diseases of the inner ear (Gloddek B. et al., Ann N Y Acad Sci 1997 Dec 29; 830:266).

Examples of autoimmune systemic diseases include, but are not limited to, systemic lupus erythematosus (Erikson J. et al., Immunol Res 1998; 17 (1-2):49) and systemic sclerosis (Renaudineau Y. et al., Clin Diagn Lab Immunol. 1999 Mar;6 (2):156); Chan OT. et al., Immunol Rev 1999 Jun;169:107).

### Infectious diseases

Examples of infectious diseases include, but are not limited to, chronic infectious diseases, subacute infectious diseases, acute infectious diseases, viral diseases, bacterial diseases, protozoan diseases, parasitic diseases, fungal diseases, mycoplasma diseases and prion diseases.

### Graft rejection diseases

Examples of diseases associated with transplantation of a graft include, but are not limited to, graft rejection, chronic graft rejection, subacute graft rejection, hyperacute graft rejection, acute graft rejection and graft versus host disease.

### Allergic diseases

Examples of allergic diseases include, but are not limited to, asthma, hives, urticaria, pollen allergy, dust mite allergy, venom allergy, cosmetics allergy, latex allergy, chemical allergy, drug allergy, insect bite allergy, animal dander allergy, stinging plant allergy, poison ivy allergy and food allergy.

### Cancerous diseases

Examples of cancer include but are not limited to carcinoma, lymphoma, blastoma, sarcoma, and leukemia. Particular examples of cancerous diseases but are not limited to: Myeloid leukemia such as Chronic myelogenous leukemia. Acute myelogenous leukemia with maturation. Acute promyelocytic leukemia, Acute nonlymphocytic leukemia with increased basophils, Acute monocytic leukemia. Acute myelomonocytic leukemia with eosinophilia; Malignant lymphoma, such as Birkitt's Non-Hodgkin's; Lymphoctyic leukemia, such as Acute lumphoblastic leukemia. Chronic lymphocytic leukemia; Myeloproliferative diseases, such as Solid tumors Benign Meningioma, Mixed tumors of salivary gland, Colonic adenomas; Adenocarcinomas, such as Small cell lung cancer, Kidney, Uterus, Prostate, Bladder, Ovary, Colon, Sarcomas, Liposarcoma, myxoid, Synovial sarcoma, Rhabdomyosarcoma (alveolar), Extraskeletel myxoid chonodrosarcoma, Ewing's tumor; other include Testicular and ovarian dysgerminoma, Retinoblastoma, Wilms' tumor, Neuroblastoma, Malignant melanoma, Mesothelioma, breast, skin, prostate, and ovarian.

Other diseases envisaged by the instant application include hormone and growth factor deficiencies; dwarfsism, organ (e.g., renal) failure (EPO) deficiencies and others.

As mentioned the above teachings can also be used for diagnostic applications. Thus, the recombinant protein may be a diagnostic protein which may be able of accumulating in a target organ(s) and may further comprise a detectable label (e.g., GFP), suitable for in vivo imaging.

An example of a diagnostic protein/reagent according to the disclosure is an antibody or an antigen binding fragment thereof. Antibodies may be double chain or single chain.

Additional objects, advantages, and novel features of the present disclosure will become apparent to one ordinarily skilled in the art upon examination of the following examples, which are not intended to be limiting. Additionally, each of the various aspects and aspects of the present disclosure as delineated hereinabove and as claimed in the claims section below finds experimental support in the following examples.

### EXAMPLES

Reference is now made to the following examples, which together with the above descriptions; illustrate the disclosure in a non limiting fashion.

Generally, the nomenclature used herein and the laboratory procedures utilized in the present disclosure include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### GCD accumulation in mouse liver orally administered with carrot cell culture expressing same

### Animals, Materials and Experimental Procedures

***Mice:*** BALB/C female mice 7-8 weeks.

### Plant cell preparation

***Construction of the expression plasmid** -* The cDNA encoding GCD (ATTC clone #65696, SEQ ID NOs. 3-4) was sub-cloned into a plasmid containing the ER-targeting signal from the *Arabidopsis thaliana* basic endochitinase gene, and the vacuolar targeting signal from tobacco chitinase A. At the 5' end of the open reading frame, the plasmid contained the 35S promoter from Cauliflower Mosaic Virus followed by the Tobacco Mosaic Virus (TMV) omega translational enhancer element. At the 3' end, the octopine synthase terminator sequence from *Agrobacterium tumefaciens* was inserted. The cassette was removed from the intermediate vector and ligated into a binary vector. Kanamycine resistance was conferred by the NPTII gene driven by the nos promoter.

***Transformation and isolation of carrot cells** -* Carrot cell suspension cultures were transformed using *Agrobacterium.* Briefly, *Agrobacteria* were transformed with the above GCD containing vector by electroporation, and selected using 30 mg/ml paromomycin. Carrot cells were transformed with *Agrobacteria,* and selected using 60 mg/ml of paromomycin in liquid media. Transformed carrot cells were plated on solid selection media, and calli were allowed to form from individual cells. High protein-expressing lines were identified and selected. Calli were further expanded and transferred to liquid media

***Determination of GCD expression by Western blotting** -* For Western blotting, protein extracts were separated by SDS-PAGE, transferred to a nitrocellulose membrane (Amersham Life Science), and GCD detected using anti-GCD antibodies (diluted 1:6500) and a peroxidase-conjugated goat anti-rabbit HRP secondary antibody (diluted 1:15,000, Sigma).

***Up-scaling in bioreactors** -* Suspension cultures of selected calli were cultured in Murashige and Skoog (MS) liquid medium containing 4.4 g/l MSD medium (Duchefa, Holland), 9.9 mg/l thiamin HCl (Duchefa, Holland), 30 g/l sucrose, and 0.1 mg/l 2,4-dichloro phenoxyacetic acid (Sigma). Suspension cell cultures were cultivated in shaking Erlenmeyer flasks used for inoculation of 10 liter polyethelenee bioreactors, followed by up-scaling to 100 liter polyethylene bioreactors. The genetically modified cells were cultivated for repeated growth cycles in the bioreactor.

***Oral administration** -* Mice were starved over night (14-16 hr) and then given plant cell material placed inside the animal cage. Following two hours, the plant material was removed and the animals were given their normal diet. Animals were sacrificed after an additional 1, 2, 4 and 18 hours. The liver from each animal was removed and frozen in liquid nitrogen and stored at -70 °C until analysis.

***Preparation of liver tissue samples:*** Each liver tissue sample was washed with 0.9 % NaCl and homogenized with homogenization buffer (60 mM phosphate citrate,1.5 % Triton X-100, 1mM PMSF) 5ml buffer per gram tissue using a ULTRA-TURRAX T 25 basic IKA-WERKE homogenizer at low speed (11,000-13,000 1/min) for 45-60 seconds, on ice. Samples were centrifuged at 10,000 g for 10 minutes at 4 °C. The supernatant was collected and divided to aliquots and frozen at -70 °C for future analysis.

***In vitro glycosidase activity assay**:* Enzymatic activity of prGCD was determined using p-nitrophenyl-β-D-glucopyranoside (Sigma) as a substrate. Assay buffer contained 60 mM phosphate-citrate buffer pH=5.5, 0.15 % Triton X-100, 0.125 % sodium taurocholate. Assay was preformed at three dilutions in total volume of 5.5 ml using 30, 12, or 6 microliter of sample, incubated with assay buffer and substrate added to final concentration of 4mM. Every 15 min, 970 microliter of each sample were removed and 30 microliter of 5N NaOH was added to each sample. Activity was measured by the rate of product (p-nitrophenyl; pNP) formation, detected by absorbance at 401 nm (Friedman, 1999).

### Results

GCD-expressing carrot cultures were used in order to test accumulation in a target organ of a recombinant protein generated and administered according to the teachings of the present disclosure. As is evident from Figure 1, peak of GCD activity in the liver was seen following 2 hours of feeding (30 % increase in enzymatic activity). This activity reverted to normal levels 4 hours following feeding.

### EXAMPLE 2

### Oral delivery of plant recombinant growth hormone (hGH) to hypophysectomized rats

The ability of plant cells to systemically deliver recombinant hGH through the GI tract was examined in hypophysectomized rats which do not express endogenous growth hormone, rendering analysis of hGH levels more simple and accurate.

### Experimental Procedures

***Plant cell preparation** -* Tobacco BY-2 cells producing hGH were produced using an inducible RNA Dependent RNA Polymerase enhanced stable cell expression system. The system was built into two plasmids. The first plasmid carried the repressor- the LacI gene under the control of the 35S promoter and a hygromicin selection followed by an IRES (internal ribosome integration site). In the second plasmid, the plant codon optimized-hGH gene (SEQ ID NOs. 1-2) flanked by the native leader and the ER retention signal was cloned under the control of a subgenomic promoter of an inducible RNA Dependent RNA Polymerase. In addition the plasmid contained the RNA Dependent RNA Polymerase (from TVCV-tobacco vein clearing virus) an IRES-NPTII selective marker gene and the LacI binding site blocking the viral replication machinery. 20mM IPTG was added to the cells carrying both plasmids, inducing GH expression. Expression levels ranged between 50-700 µg/gram fresh weight.

Determination of recombinant hGH uptake was effected using ELISA for hGH in serum and organs of rats fed with hGH (GenBank Accession No. P01241).

Two groups of n=4 rats - hypophysectomized Sprague Dawley were fed with (1) native BY2 cells (- Tobacco BY-2 Cells, Edited by Nagata, Toshiyuki; Hasezawa, Seiichiro; Inz', Dirk Springer 2004); and (2) BY2 cells expressing GH (By2 Icon repressor (B5.1+21450) GHs Nat ER line 18 induced with IPTG.

The rats were fed ad-libitum for 24 hours plant cells only (no rat chow). Following 24 hours the rats were anesthetized and blood collected, after which the rats were sacrificed and their organs collected for hGH uptake measurement. Organs collected included muscle, liver, and spleen. The serum was separated from the blood. All organs were kept in -70° C until analysis. hGH levels in serum and organs was measured by ELISA (Human growth hormone enzyme immunoassay test kit: BioCheck, Inc. catalog number BC-1033).

### Results

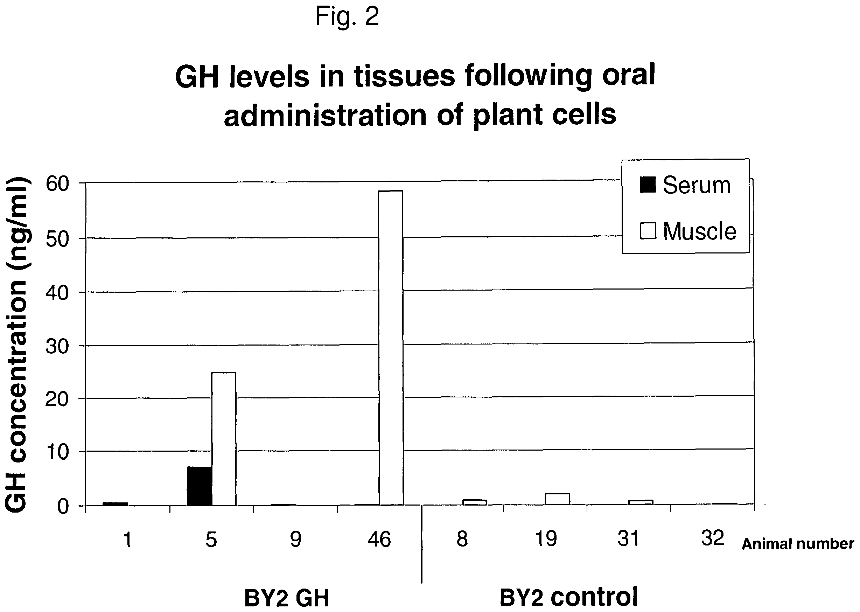

One out of the 4 rats fed with BY2 cells expressing GH exhibited significantly elevated levels of hGH in the serum. Two of the 4 rats fed with BY2 cells expressing GH had significantly elevated levels of GH in their muscle. All other rats (control, fed with BY2 naive cells) had no detectable GH levels in the serum or muscle, in accordance with their hypophysectomized phenotype. Results are shown in Figure 2 and summarized in Table 1 below.

**Table 1**

| **hGH levels in tissue and serum (AVG results of 2 independent tests).** | | | | | |
|---|---|---|---|---|---|
| **Treatment** | **Rat#** | **Serum (ng/ml)** | **Muscle (ng/mg)** | **Spleen (ng/mg)** | **Liver (ng/mg)** |
| BY2 GH | **1** | 0.572 | 0.000 | 0 | 0 |
| | **5** | **7.133** | **24.784** | 0 | 0 |
| | **9** | 0.219 | 0.000 | 0 | 0 |
| | **46** | 0.177 | **58.260** | 0 | 0 |
| BY2- | **8** | 0.012 | 1.010 | N/A | 0 |
| | **19** | 0.000 | 2.055 | N/A | 0 |
| | **31** | 0.000 | 0.760 | N/A | N/A |
| | **32** | 0.003 | 0.0.265 | N/A | N/A |

### EXAMPLE 3

### Oral delivery of plant recombinant hGCD to rats

The ability of plant cells to deliver recombinant hGCD through the digestive system for uptake of the recombinant protein into the body was examined as described in Example 1 above.

### Animals, Materials and Experimental Procedures

***Rats:*** Twelve Sprague Dawley female rats 10-11 weeks were used.

***Oral administration**:* Rats were starved over night (14-16 hr) and then given plant cell material (ad-libitum, 10 gr/rat) placed inside the animal cage. Following two hours, the plant material was removed and the animals were given their normal diet. Animals were sacrificed (3 animals at each time-point) after an additional 1, 2, 4 and 24 hours. The liver and spleen from each animal was removed and frozen in liquid nitrogen and stored at -70 °C until analysis.

### Preparation of of spleen and liver tissue: As described in Example 1.

### Results

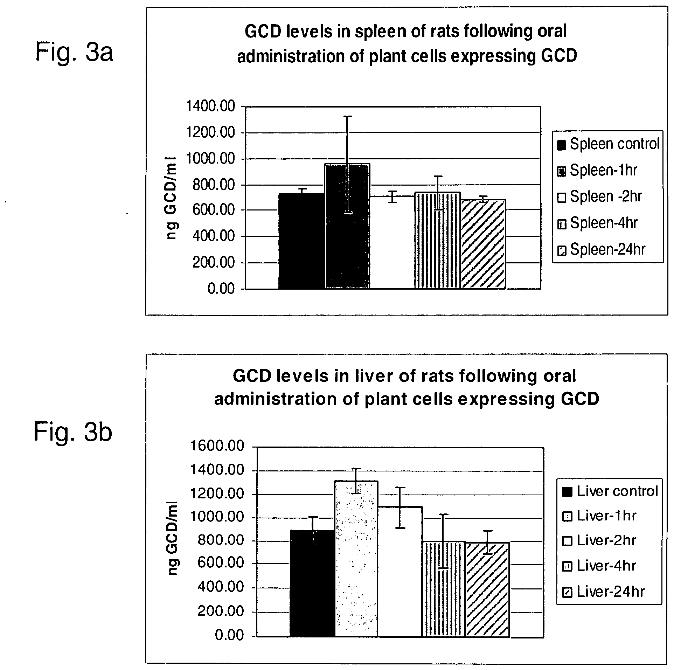

GCD-expressing carrot cultures were used in order to test accumulation in target organs of a recombinant protein generated and orally administered according to the teachings of the present disclosure. As is evident from Figure 3a and 3b, an increase in GCD activity was observed in the liver and spleen tissues with the peak of GCD activity seen following 1 hour after of feeding (Spleen-26 %, Liver-44 % increase in enzymatic activity). This activity reverted to normal levels 4 hours following feeding.

### EXAMPLE 4

### Oral delivery of plant recombinant FSH to hypophysectomized rats

The ability of plant cells to deliver FSH to the blood through the GI tract was examined following a single oral (PO) administration of plant cell material.

### Experimental Procedures

### Plant cell preparation

***Construction of the expression plasmid** -* The DNA encoding FSH α and β chains with their native signals were sub-cloned into a plasmid containing the 35S promoter from Cauliflower Mosaic Virus followed by the Tobacco Mosaic Virus (TMV) omega translational enhancer element. At the 3' end, the octopine synthase terminator sequence from *Agrobacterium tumefaciens* was inserted. The cassette was removed from the intermediate vector and ligated into the binary vector. The FSH P chain had the Kanamycine resistance conferred by the NPTII gene driven by the nos promoter, and the FSH α chain had the Hygromicine resistance conferred by the aphIII gene driven by the nos promoter.

***Transformation and isolation of carrot cells** -* Carrot cell suspension cultures were transformed using *Agrobacterium.* Briefly, *Agrobacteria* were transformed with the above FSH β vector by electroporation, and selected using 30 mg/ml paromomycin. Carrot cells were transformed with *Agrobacteria,* and selected using 60 mg/ml of paromomycin in liquid media. Transformed carrot cells were plated on solid selection media, and calli were allowed to form from individual cells. High protein-expressing lines were identified and selected. Calli were further expanded and transferred to liquid media. The cells were then re-transformed with the plasmid carrying the FSH α chain and selected as above using 100mg/ml hyromicine. The best expressing line was selected using western blots and ELISA assays to evaluate the expression levels. These were 1-20µg /gFW

***Animals and oral administration** -* Three 11 week old rats (200 gr) were administered 10 ml/kg plant carrot cell mash by oral gavage. Blood samples were taken predose and at 10, 20, 30, 60, 120 and 240 min following administration and FSH concentrations were measured by ELISA using a kit from BioCheck Inc.

### Results

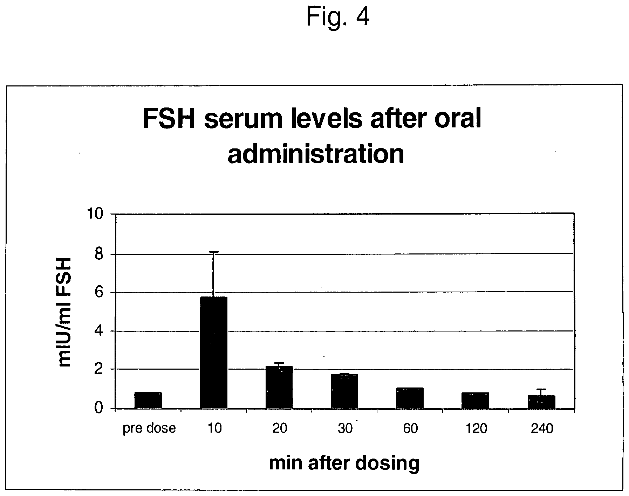

As shown in Figure 4, FSH serum levels increased by at least 5 fold 10 minutes following administration after which they revered to basal.

### EXAMPLE 5

### Lypholyzed carrot cells express maintain intact GCD levels and activity

The ability of the plant cells of the present disclosure to maintain activity following dehydration was determined.

### Experimental Procedures

A hundred grams of carrot cells expressing GCD produced as described in Example 1 above were lypholyzed and tested for specific protein expression and activity (see Examples 1 and 3 above).

***Lysate preparation** -* 50 mg dry cells were extracted with 1 ml extraction buffer pH=7.2 (20mM phosphate buffer pH=7.2, 20mM EDTA, 20mM L-Ascorbic acid, 1% Triton X-100).

***Determination of GCD expression by Western blotting** -* For Western blotting, protein extracts were separated by SDS-PAGE, transferred to a nitrocellulose membrane (Amersham Life Science), and GCD detected using the anti-GCD antibodies (diluted 1:6500) and a peroxidase-conjugated goat anti-rabbit HRP secondary antibody (diluted 1:15,000) (Sigma).

### Results

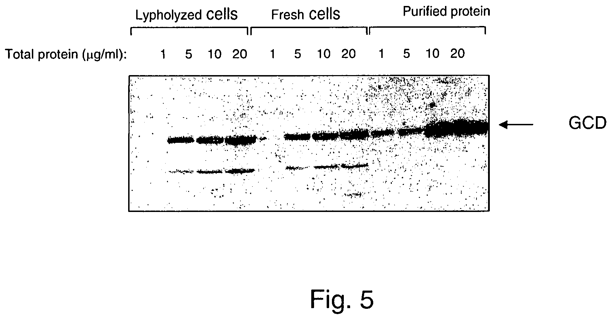

Figure 5 shows GCD expression in lypholyzed plant cells versus fresh cells. Protein load was based on total protein measurement by Bradford. As shown the levels of GCD expression in the dry preparation were similar to those of the fresh preparation. Total protein in the lysates was measured and the amount of active GCD in both lypholyzed and fresh cell lysates was evaluated. Results are shown in Table 2, below.

**Table 2**

| | Total protein mg/ml | GCD concentration µg/ml |
|---|---|---|
| Lypholyzed cells | 2.582 | 2.010 |
| Fresh cells | 2.799 | 2.505 |

Thus it is evident that dry preparations of the present disclosure maintain their biological activities.

Altogether, the above results demonstrate for the first time the ability of non-isolated plant expressed recombinant protein to move across the GI tract and accumulate in internal organs.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A unit dosage form for systemic delivery of a biologically active recombinant biomolecule to a subject, the unit dosage form comprising a therapeutically effective amount of dehydrated isolated plant cells of the BY2 tobacco cell line, said isolated plant cells expressing the recombinant biomolecule.

2. The unit dosage form of claim 1, formulated for oral administration.

3. The unit dosage form of claim 1, formulated for mucosal administration.

4. The unit dosage form of any of claims 1, wherein said recombinant biomolecule comprises a recombinant polypeptide.

5. The unit dosage form of claim 4, wherein said recombinant polypeptide comprises a lysosomal enzyme.

6. The unit dosage form of claim 4, wherein said recombinant polypeptide comprises an alpha galactosidase.

7. The unit dosage form of claim 1, wherein said recombinant biomolecule is selected from the group consisting of a chimeric protein, an enzyme, a growth factor, a protease, a lysosomal enzyme, an extra-cellular matrix protein, an infectious viral protein, an antisense oligonucleotide, a dsRNA and a ribozyme.

8. The unit dosage form of claim 2, selected from the group consisting of a tablet, a pill, a dragee, a capsule, a liquid, a gel, a syrup, a slurry, a suspension.

9. The unit dosage form of claim 3, being a food.

10. Isolated plant cells of the BY2 tobacco cell line for use in the treatment of Fabry disease, said isolated plant cells expressing a human alpha galactosidase.

11. Isolated plant cells of the BY2 tobacco cell line for use in the treatment of dwarfism, said isolated plant cells expressing a human growth hormone.

12. The cells for the use of claim 10 or 11, formulated for oral administration.

13. The cells for the use of claim 10 or 11, formulated for mucosal administration.

14. The cells for the use of claim 10 or 11, wherein said isolated plant cells comprise dehydrated plant cells.

## Patentansprüche

1. Einheitsdarteichungsform zur systemischen Abgabe eines biologisch aktiven rekombinanten Biomoleküls an ein Subjekt, wobei die Einheitsdameichungsform eine therapeutisch wirksame Menge von dehydrierten isolierten Pflanzenzellen der BY2-Tabakzelllinie umfasst, wobei die isolierten Pflanzenzellen das rekombinante Biomolekül exprimieren.

2. Einheitsdarreichungsform nach Anspruch 1, die für eine orale Verabreichung formuliert ist.

3. Einheitsdarreichungsform nach Anspruch 1, die für eine Schleimhautverabreichung formuliert ist.

4. Einheitsdarreichungsform nach einem der Ansprüche 1, wobei das rekombinante Biomolekül ein rekombinantes Polypeptid umfasst.

5. Einheitsdarreichungsform nach Anspruch 4, wobei das rekombinante Polypeptid ein lysosomales Enzym umfasst.

6. Einheitsdarreichungsform nach Anspruch 4, wobei das rekombinante Polypeptid eine Alpha-Galactosidase umfasst.

7. Einheitsdarreichungsform nach Anspruch 1, wobei das rekombinante Biomolekül aus der Gruppe ausgewählt ist, bestehend aus einem chimären Protein, einem Enzym, einem Wachstumsfaktor, einer Protease, einem lysosomalen Enzym, einem extrazellulären Matrixprotein, einem infektiösen Virusprotein, einem Antisense-Oligonukleotid, einer dsRNA und einem Ribozym.

8. Einheitsdarreichungsform nach Anspruch 2, ausgewählt aus der Gruppe, bestehend aus einer Tablette, einer Pille, einem Dragee, einer Kapsel, einer Flüssigkeit, einem Gel, einem Sirup, einer Aufschlämmung, einer Suspension.

9. Einheitsdarreichungsform nach Anspruch 3, die ein Lebensmittel ist.

10. Isolierte Pflanzenzellen der BY2-Tabakzelllinie zur Verwendung bei der Behandlung von Morbus Fabry, wobei die isolierten Pflanzenzellen eine humane Alpha-Galactosidase exprimieren.

11. Isolierte Pflanzenzellen der BY2-Tabakzelllinie zur Verwendung bei der Behandlung von Kleinwuchs, wobei die isolierten Pflanzenzellen ein humanes Wachstumshormon exprimieren.

12. Zellen zur Verwendung nach Anspruch 10 oder 11, die für eine orale Verabreichung formuliert sind.

13. Zellen zur Verwendung nach Anspruch 10 der 11, die für eine Schleimhautverabreichung formuliert sind.

14. Zellen zur Verwendung nach Anspruch 10 oder 11, wobei die isolierten Pflanzenzellen dehydrierte Pflanzenzellen umfassen.

## Revendications

1. Forme galénique unitaire pour l'administration systémique d'une biomolécule recombinante biologiquement active à un sujet, la forme galénique unitaire comprenant une quantité thérapeutiquement efficace de cellules végétales isolées déshydratées de la lignée cellulaire de tabac BY2, lesdites cellules végétales isolées exprimant la biomolécule recombinante.

2. Forme galénique unitaire selon la revendication 1, formulée pour une administration orale.

3. Forme galénique unitaire selon la revendication 1, formulée pour une administration par les muqueuses.

4. Forme galénique unitaire selon l'une quelconque des revendications 1, dans laquelle ladite biomolécule recombinante comprend un polypeptide recombinant.

5. Forme galénique unitaire selon la revendication 4, dans laquelle ledit polypeptide recombinant comprend une enzyme lysosomale.

6. Forme galénique unitaire selon la revendication 4, dans laquelle ledit polypeptide recombinant comprend un alpha galactosidase.

7. Forme galénique unitaire selon la revendication 1, dans laquelle ladite biomolécule recombinante est choisie dans le groupe constitué d'une protéine chimérique, d'une enzyme, d'un facteur de croissance, d'une protéase, d'une enzyme lysosomale, d'une protéine de la matrice extracellulaire, d'une protéine virale infectieuse, d'un oligonucléotide antisens, d'un ARN bicaténaire et d'un ribozyme.

8. Forme galénique unitaire selon la revendication 2, choisie dans le groupe constitué d'un comprimé, d'une pilule, d'une dragée, d'une capsule, d'un liquide, d'un gel, d'un sirop, d'une pâte, d'une suspension.

9. Forme galénique unitaire selon la revendication 3, étant un aliment.

10. Cellules végétales isolées de la lignée cellulaire de tabac BY2 pour utilisation dans le traitement de la maladie de Fabry, lesdites cellules végétales isolées exprimant un alpha galactosidase humain.

11. Cellules végétales isolées de la lignée cellulaire de tabac BY2 pour utilisation dans le traitement du nanisme, lesdites cellules végétales isolées exprimant une hormone de croissance humaine.

12. Cellules pour l'utilisation selon la revendication 10 ou 11, formulée pour une administration orale.

13. Cellules pour l'utilisation selon la revendication 10 ou 11, formulée pour une administration par les muqueuses.

14. Cellules pour l'utilisation selon la revendication 10 ou 11, dans lesquelles lesdites cellules végétales isolées comprend des cellules végétales déshydratées.
